# EUROPEAN PATENT SPECIFICATION

(11) **EP 2 935 205 B1**
(45) Date of publication and mention of the grant of the patent: **01.03.2017**
(21) Application number: 13818745.5
(22) Date of filing: 20.12.2013
(51) Int. Cl.: C07C 233/51, C12P 13/04

(54) **AN ENZYMATIC ROUTE FOR THE PREPARATION OF CHIRAL GAMMA-ARYL-BETA-AMINOBUTYRIC ACID DERIVATIVES**
ENZYMATISCHER WEG FÜR DIE HERSTELLUNG VON CHIRALEN GAMMA-ARYL-BETA-AMINOBUTYLSÄUREDERIVATE
ITINÉRAIRE ENZYMATIQUE POUR LA PRÉPARATION DE DÉRIVÉS D'ACIDE CHIRAL GAMMA-ARYL-BETA-AMINOBUTYRIQUE

(30) Priority: 21.12.2012 EP 12198811
(43) Date of publication of application: 28.10.2015
(73) Proprietor: LEK Pharmaceuticals d.d., 1526 Ljubljana (SI)
(72) Inventor: STARCEVIC, Stefan, 1526 Ljubljana (SI); MRAK, Peter, 1526 Ljubljana (SI); KOPITAR, Gregor, 1526 Ljubljana (SI)
(74) Representative: Prüfer & Partner mbB Patentanwälte · Rechtsanwälte
(86) International application number: PCT/EP2013/077728
(87) International publication number: WO 2014/096375

(56) References cited:
- EP-A1- 1 604 975
- TASNÁDI GÁBOR ET AL: "Improved enzymatic syntheses of valuable beta-arylalkyl-beta-amino acid enantiomers.", ORGANIC & BIOMOLECULAR CHEMISTRY 21 FEB 2010, vol. 8, no. 4, 21 February 2010 (2010-02-21), pages 793-799, XP002693104, ISSN: 1477-0539
- LILJEBLAD A ET AL: "Biocatalysis as a profound tool in the preparation of highly enantiopure beta-amino acids", TETRAHEDRON, ELSEVIER SCIENCE PUBLISHERS, AMSTERDAM, NL, vol. 62, no. 25, 19 June 2006 (2006-06-19) , pages 5831-5854, XP025002086, ISSN: 0040-4020, DOI: 10.1016/J.TET.2006.03.109 [retrieved on 2006-06-19]

## Description

### Field of the Invention

The present invention relates to the field of organic chemistry, more specifically to a process for preparing chiral γ-aryl-β-aminobutyric acid derivatives. Such compounds are useful as key structure framework of modern drug chemistry and especially of antidiabetic agents.

### Background of the Invention

β-Amino acids are of interest in the preparation of active pharmaceutical ingredients (APIs). The β-amino acid moiety in APIs of interest is normally part of a complex whole structure. Complexity is typically enhanced when considering a chiral center at the β-position of the β-aminobutyryl group and the general desire to obtain enantiopure compounds.

A particularly interesting class of APIs having β-amino acid structural moieties are dipeptidyl peptidase-4 (DPP-4) inhibitors which act as antidiabetic agents. DPP-4 inhibitors are oral antidiabetic drugs which reduce glucose blood levels by a new mechanism of action in which the DPP-4 inhibitors ("gliptins") inhibit inactivation of glucagon-like peptide (GLP), which stimulate insulin secretion. The benefit of these medicines lies in their lower side-effects (e.g., less hypoglycemia, less weight gain) and in the control of blood glucose values. It can be used for treatment of diabetes mellitus type 2.

The first member of the novel pharmacological group is sitagliptin (compound of formula la) which is chemically (*R*)-3-amino-1-(3-(trifluoromethyl)-5,6-dihydro-[1,2,4]triazolo[4,3-a]pyrazin-7(8H)-yl)-4-(2,4,5-trifluorophenyl)butan-1-one and which has the following structural formula 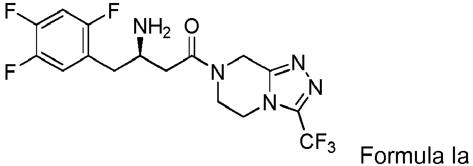 including a β-amino acid part.

However, an inclusion of a β-amino acid framework into a more complex molecule remains a permanent challenge for industrial production.

This is well reflected in the literature for the synthesis of sitagliptin. Several methods are described how to introduce the β-amino acid structure into the molecule of sitagliptin. The first synthesis of sitagliptin molecule disclosed in WO 03/004498 uses an unusual chiral dihydropyrazine promoter, diazomethane and silver salts, which compounds are unacceptable reagents for industrial synthesis. The synthetic pathway of WO 03/004498 is depicted in Scheme 1.

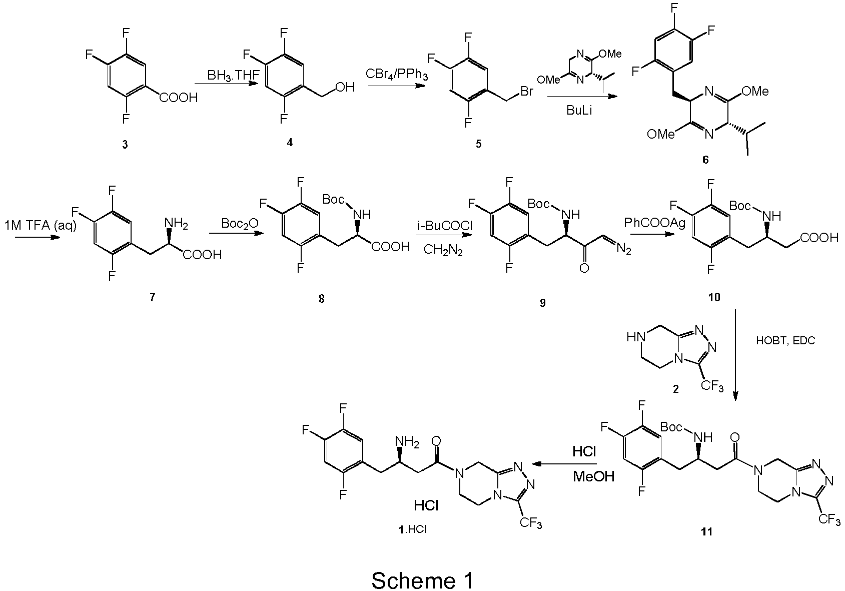

Since then, several trials to improve this unacceptable method have been published in literature. In general, regarding the structure of sitagliptin, which is composed from the β-amino acid part and the heterocyclic part, synthetic routes can be divided in two approaches.

In the first general approach, a heterocycle is coupled to the system in earlier steps of the synthesis, while the desired configuration of β-amino acid part is constructed later. This approach seems less feasible, because typically, it is better to couple more complicated and more expensive parts of molecule in last steps. A typical example of this approach is shown in Scheme 2.

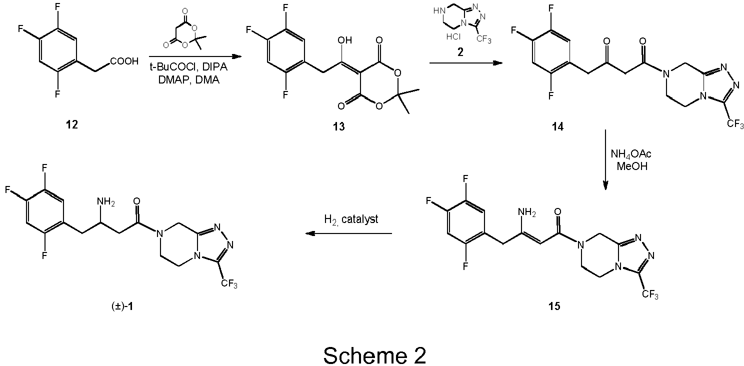

There is no good method for non-chromatographical chiral resolution of final compound **1** (WO 09/084024), so the resolution is performed by enantioselective reduction of **15** to **1.** Such enantioselective hydrogenation of β-enamino acid derivatives requires expensive precious metal catalysts, such as rhodium (WO 03/004498, Tetrahedron Asymmetry 17, 205 (2006*))* or ruthenium (WO 09/064476) and expensive ligands, such as ferrocenyl diphosphine ligands - JOSIPHOS catalysts (WO 04/085378, WO 05/097733, WO 06/081151, WO 11/113399, J. Am. Chem. Soc., 126, 9918 (2004)).

Another option is a derivatization of the amino group with a chiral group. Chiral resolution is then achieved by hydrogenation with a cheaper achiral catalyst, by crystallisation of diastereomeric mixtures or by combination of both methods, as depicted in Scheme 3 (WO 04/085661, WO 09/085990, WO 11/025932, WO 11/060213, WO 11/142825). These methods suffer from considerable loss of material in order to obtain pharmaceutical grade chiral purity.

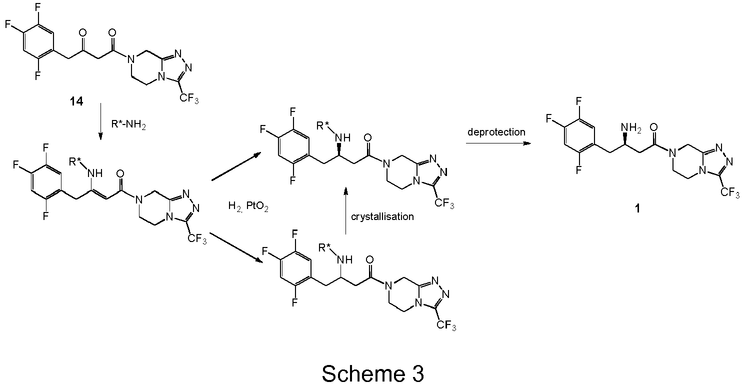

In the second general approach, a heterocycle is coupled to the β-amino acid in later steps. The corresponding γ-aryl-β-amino acids are readily available from the corresponding β-keto acids, prepared from acetic acids and malonic derivatives (WO 09/064476, WO 10/122578, WO 10/131025, WO 11/127794). Unfortunately, the amino group needs protection before coupling with the heterocycle in order to eliminate side reactions. As can be gathered from Scheme 4, the protection/deprotection scenario considerably prolongs the synthesis of antidiabetic agents (Scheme 4).

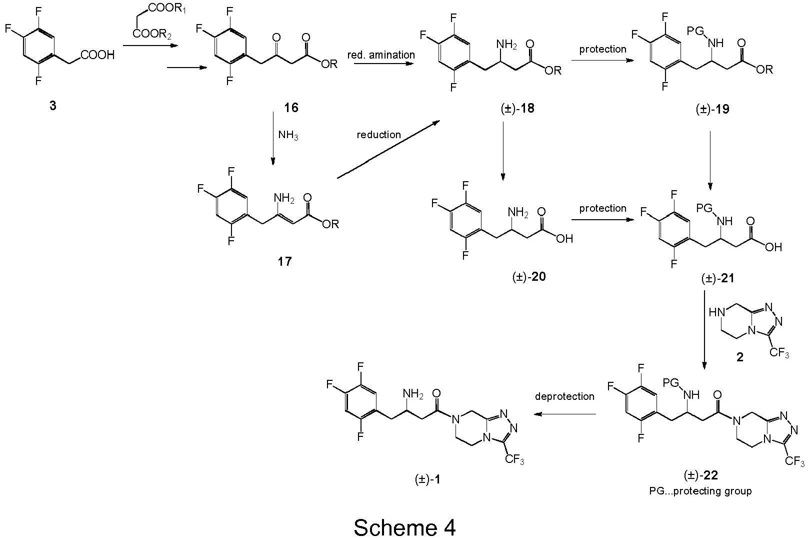

There are several methods in literature how to prepare enantiomerically enriched or pure intermediates **18, 19, 20,** and **21,** such as by enantioselective reduction of **17** (WO 09/064476, WO 10/078440), by introducing chiral protecting groups with further diastereoselective crystallization (CN 102126976), by crystallization of diastereomeric salt of compounds (±)-**18**, (±)-**19**, (±)-**20**, or (±)-**21** with chiral acids (WO 10/122578, WO 10/131025, J. Chem. Res. (4), 230 (2010)), or by introduction of a chiral center via natural source, such as aspartic acid derivatives (WO 11/035725, WO 11/116686A2, CN 102093245, CN 10212697). or by enzymatic approach.

A biocatalytic approach represents a promising method for preparation chiral compound in many cases, but the compound **15** in Scheme 2 is less suitable for routine chiral enzymatic approaches due to bulky unnatural derivatisation of carboxylic part. The problem was solved by mutation of the natural enzyme (Science, 329, 305 (2010*)*), but such enzyme is not available for a routine chemist. In addition this approach uses enzymatic conversion in the last step of the synthesis, potentially resulting in additional unwanted purification steps due to residual protein carry-over.

Yet another option of biocatalytic creation a chiral center was done by selective reduction of β-keto acid derivatives (WO 09/045507), but further transformation of the obtained chiral hydroxyl intermediates to final sitagliptin precursors via azetidinone intermediates was laborious, as can be gathered from Scheme 5 (WO 10/122578, EP 2397141).

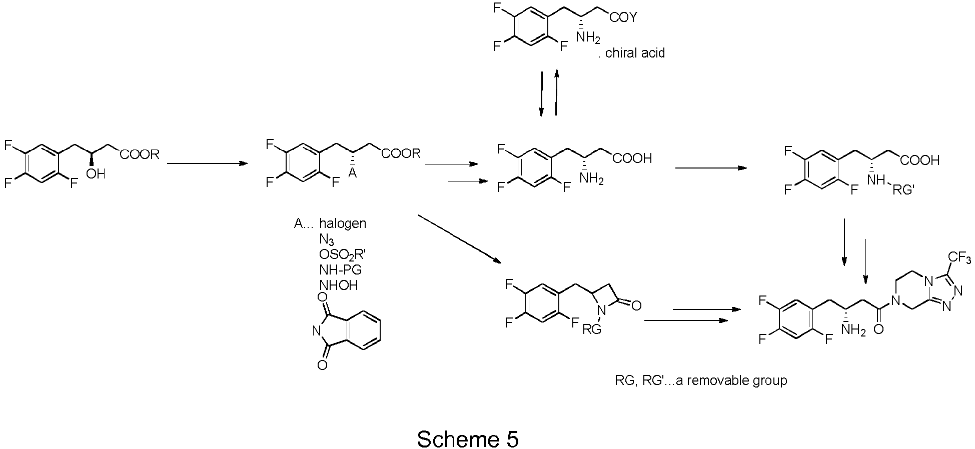

Therefore, there is still a need to find, and thus it was an object of the present invention to provide, a process that is improved in terms of ease, cheapness and suitability of industrial applications for preparing chiral γ-aryl-β-aminobutyric acid derivatives, in particular those having a desired enantiomeric excess, respectively representing valuable key intermediates for the preparation of pharmaceutically active agents or representing a pharmaceutically active agent such as dipeptidyl peptidase-4 (DPP-4) inhibitors, e.g. sitagliptin.

### Summary of the Invention

The present invention provides the following items:
(1) An enantioselective process for the preparation of a compound of Formula (*R*)-II: 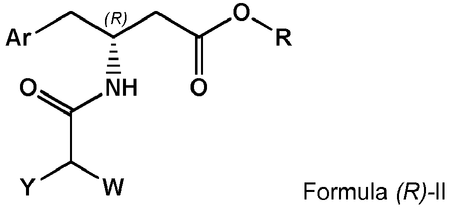 wherein
   Ar is unsubstituted or substituted C₆-C₁₂-aryl, R is H or C₁-C₄-alkyl and
   W denotes unsubstituted or substituted C₆-C₁₂-aryl, furanyl or thienyl and Y independently denotes hydrogen, hydroxy, amino, chloro, bromo or methyl, or
   W denotes unsubstituted or substituted C₆-C₁₂-aryloxy, C₅-C₁₂-heterocycle or -heteroaryl, and Y is hydrogen,
      comprising
      (i) N-acylation of γ-aryl-β-aminobutyric acid or acid derivative of Formula-III, 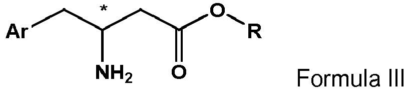 wherein Ar and R are defined as above
         with an activated acid derivative of the compound of Formula IV 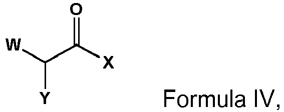 wherein Y and W are defined as above and X is a leaving group, in a solvent and a presence of a base to afford substituted acetylated γ-aryl-β-aminobutyric acid compound of Formula II 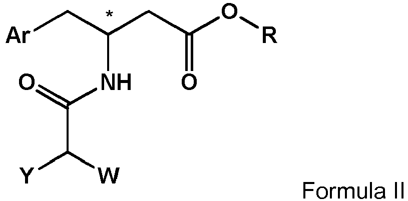
      (ii) kinetic resolution of the compound of Formula II with penicillin amidase enzyme in an aqueous medium to obtain a compound of Formula II which is enantiomerically enriched in the *(R)*-enantiomer, denoted (*R*)-II (relative to the corresponding *(S)-*enantiomer of compound of Formula II) and enantiomerically enriched compound of Formula *(S)*-III 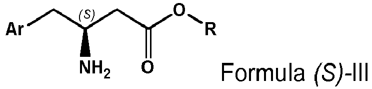 wherein Ar and R are defined as above, and
      (iii) obtaining the compound of Formula *(R)*-II.

   The designation "*" in the structural formulae of compounds of formula II and III indicates a chiral center. The term "chiral center" as used herein means a tetrahedral carbon atom having four different substituents attached thereto. The arrangement of these different substituents around the asymmetric atom determines its optical activity which is denoted in the art by the terms S or R and D or L respectively. Herein, the designations "R" and "S" will be used, indicating the absolute configuration at chiral center(s) of the respective compounds, determined by means of the known Cahn Ingold Prelog convention (CIP-convention).
   Preferably, γ-aryl-β-aminobutyric acid derivative of Formula-III provided for use in step (i) is racemic mixture or a mixture of enantiomers with poor excess of one enantiomer.
   Further, in step (ii) the compound of Formula II is preferably obtained in the form of in highly enantiomerically enriched *(R)*-enantiomer, and more preferably is even obtained in a substantially enantiopure form.
   The term "C₆-C₁₂-aryl" as defined herein includes reference to an aromatic ring system comprising 6, 7, 8, 9, 10, 11 or 12 ring carbon atoms. The term "C₆-C₁₂-aryl" in particular includes, without being limited to, phenyl, 1-naphtyl, 2-naphtyl, fluorenyl, azulenyl, indenyl, or anthryl .
   The term "C₅-C₁₂-heterocycle or -heteroaryl" as defined herein includes a saturated (e.g. heterocycloalkyl) or unsaturated (e.g. heteroaryl) heterocyclic ring moiety having 5, 6, 7, 8, 9, 10, 11 or 12 ring atoms, at least one of which is selected from nitrogen and oxygen. The term "heterocycle" particularly includes a 5- to 10-membered ring or ring system and more particularly a 5- or 6- or 7-membered ring, which may be saturated or unsaturated; examples thereof include oxiranyl, azirinyl, 1,2-oxathiolanyl, imidazolyl, thienyl, furyl, tetrahydrofuryl, pyranyl, thiopyranyl, thianthrenyl, isobenzofuranyl, benzofuranyl, chromenyl, 2H-pyrrolyl, pyrrolyl, pyrrolinyl, pyrrolidinyl, imidazolyl, imidazolidinyl, benzimidazolyl, pyrazolyl, pyrazinyl, pyrazolidinyl, thiazolyl, isothiazolyl, dithiazolyl, oxazolyl, isoxazolyl, pyridyl, pyrazinyl, pyrimidinyl, piperidyl, piperazinyl, pyridazinyl, morpholinyl, thiomorpholinyl, especially thiomorpholino, indolizinyl, isoindolyl, 3H-indolyl, indolyl, benzimidazolyl, cumaryl, indazolyl, triazolyl, tetrazolyl, purinyl, 4H-quinolizinyl, isoquinolyl, quinolyl, tetrahydroquinolyl, tetrahydroisoquinolyl, decahydroquinolyl, octahydroisoquinolyl, benzofuranyl, dibenzofuranyl, benzothiophenyl, dibenzothiophenyl, phthalazinyl, naphthyridinyl, quinoxalyl, quinazolinyl, quinazolinyl, cinnolinyl, pteridinyl, carbazolyl, ß-carbolinyl, phenanthridinyl, acridinyl, perimidinyl, phenanthrolinyl, furazanyl, phenoxazolyl, phenazinyl, phenothiazinyl, phenoxazinyl, ehromenyl, isochromanyl, chromanyl and the like.
   More specifically, a saturated heterocyclic moiety may have 5, 6 or 7 ring carbon atoms and 1, 2, 3, 4 or 5 ring heteroatoms selected from nitrogen and oxygen. The group may be a polycyclic ring system but more often is monocyclic, for example including azetidinyl, pyrrolidinyl, tetrahydrofuranyl, piperidinyl, oxiranyl, pyrazolidinyl, imidazolyl, indolizidinyl, piperazinyl, thiazolidinyl, morpholinyl, thiomorpholinyl, quinolinidinyl and the like. Furthermore, the "heteroaryl" may include an aromatic heterocyclic ring system having 5, 6, 7, 8, 9, 10, 11 or 12 ring atoms, at least one of which is selected from nitrogen and oxygen. The group may be a polycyclic ring system, having two or more rings, at least one of which is aromatic, but is more often monocyclic. This term includes pyrimidinyl, furanyl, benzo[b]thiophenyl, thiophenyl, pyrrolyl, imidazolyl, pyrrolidinyl, pyridinyl, benzo[b]furanyl, pyrazinyl, purinyl, indolyl, benzimidazolyl, quinolinyl, phenothiazinyl, 2-oxo-3-oxazolyl, [4,5]-phen-2-oxo-3-oxazolyl, triazinyl, phthalazinyl, oxazolyl, isoxazolyl, thiazolyl, isoindolyl, indazolyl, purinyl, isoquinolinyl, quinazolinyl, pteridinyl and the like.
   Preferred "C₅-C₁₂-heterocycle or -heteroaryl" can be selected from 1-tetrazolyl, oxazolyl, phenoxazolyl, 2-oxo-3-oxazolyl and 4,5-phen-2-oxo-3-oxazolyl.
   The term "substituted" as defined herein, means being substituted with one or more substituents which are inert in the reaction of the invention and which most distant atom is at most 5 atoms far from aryl, selected but not limited to halo, nitro, cyano, hydroxy, C₁-C₃-alkoxy, amino, mono or di(C₁-C₃-alkyl)amino, C₁-C₄-alkyl.
   The term "enantiomer" as defined herein is one component of a pair of optically active compounds (chiral compounds), which are represented herein with an asymmetric carbon center (chiral center) binding the amino group.
   The terms "racemate" or "racemic compound" as defined herein is a mixture of both enantiomers of said pair of optically active compounds in equal amounts.
   The term "enantiomeric excess" (ee) as defined herein represents a numerical value of an excess of one enantiomer compared to racemate in a mixture of both enantiomer, expresses per cent (%).
   The term "enantiomerically enriched" as defined herein represents a mixture of enantiomers with enantiomeric excess of one component.
   The terms "highly enantiomerically enriched" or "a mixture of high excess of one enantiomer" as defined herein represent a mixture of enantiomers with enantiomeric excess of one component at least 90 %, preferably at least 95 %, more preferably at least 99 %, most preferably at least 99.5 %.
   The terms "poorly enantiomerically enriched" or "a mixture of poor excess of one enantiomer" as defined herein represent a mixture of enantiomers wherein enantiomeric excess of one component does not exceed 20 %.
   The term "activated derivative" as defined herein represents a derivative with a group, which reacts easier or faster than the mother compound, in the case of the acid derivative as defined herein X is preferably selected from halo, preferably chloro, acyl, preferably C₁-C₄-alkoxy carbonyl, imidazolyl, 1-benzotriazolyloxy, di(C₁-C₄-alkyl)phosphoryloxy.
   The term "kinetic resolution" as defined herein represents an enzymatically catalyzed hydrolytic reaction in which one enantiomer of racemic starting compound reacts much slower than the opposite enantiomer. Consequently, the time point is reached, where one enantiomer of starting compound is consumed, preferably is completely consumed, while the other enantiomer of the starting compound is enriched, preferably is the predominantly or even only remaining enantiomer. The reaction herein can be stopped, and preferably is stopped, at the point where enantiomeric excess (ee) of the unreacted enantiomer of starting compound reaches at least 90 %, preferably at least 95 %, more preferably at least 99%, most preferably at least 99.5 %.
   In this way, preferably highly enantiomerically enriched and even enantiopure or substantially enantiopure compound of formula (R)-II can be beneficially obtained by means of enzymatic kinetic resolution, which is particularly favourable in the desire to provide certain pharmaceutically active agents such as dipeptidyl peptidase-4 (DPP-4) inhibitors, e.g. sitagliptin. In particular, it was surprisingly found that by means of enzymatic kinetic resolution using penicillin amidase enzyme subjected to the derivative of Formula II, the desired (R)-enantiomer of compound of formula II is less subjected to hydrolytic reaction than the corresponding (S)-enantiomer and thus the said desired (R)-enantiomer remains in higher ratio, while the other (S)-enantiomer of compound of formula II is increasingly converted into the compound of Formula (S)-III.
   Due to different characteristics of both, the desired (R)-enantiomer of compound of formula II and the compound of Formula (S)-III, these can be easily separated, e.g. by physical and/or chemical methods. By this kinetic resolution, the compound of formula (*R*)-II can, in a preferred embodiment, be provided in highly enantiomerically enriched or even substantially enantiopure form, and thus it can be dispensed with laborious and costly racemic resolution of compounds of formulae II or III, e.g. by means of conventional formation of diastereomeric salts, by chiral chromatography, or by other means.
(2) The process according to item (1), wherein in step (i) the γ-aryl-β-aminobutyric acid derivative of Formula-III is in racemic form or in form of a mixture of enantiomers with poor excess of either one of the enantiomers, and/or the derivative of Formula II is in racemic form or in form of a mixture of enantiomers with poor excess of either one of the enantiomers.
(3) The process according to item (1) or (2), wherein in step (ii) the compound of Formula II which is highly enantiomerically enriched in the (R)-enantiomer.
(4) The process according to any one of the preceding items, wherein in step (iii) the compound of Formula *(R)*-II is obtained by acidifying the aqueous medium and extracting the compound of Formula *(R)*-II from acidic aqueous medium with a water immiscible solvent, optionally subsequently removing the water immiscible solvent.
(5) The process according to any one of the preceding items, wherein Ar is selected from substituted phenyl, preferably is halo substituted phenyl, more preferably fluoro substituted phenyl, most preferably 2,4,5-trifluorophenyl.
(6) The process according to any one of the preceding items, wherein X is chloro.
(7) The process according to any one of the preceding items, wherein W is phenyl.
(8) The process according to any one of the preceding items, wherein R initially is H, or wherein the OR group in the respective compounds is converted into OH at any stage of the process.
(9) The process according to any one of the preceding items, wherein the compound of Formula *(R)*-II obtained in step (iii) is hydrolysed to obtain a compound of Formula *(R)*-III 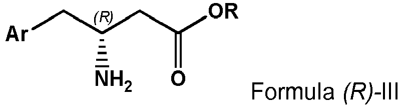 wherein Ar is as defined above, preferably is 2,4,5-trifluorophenyl.
(10) The process according to the preceding item, wherein R is H, thereby obtaining a compound of Formula *(R)*-III': 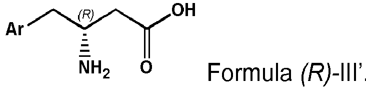
(11) The process according to any one of the preceding items, wherein enantiomerically enriched compound of Formula *(S)*-III 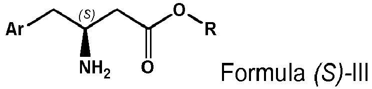 wherein Ar and R are defined as above, is used to subsequently prepare a pharmaceutically active compound.
(12) The process according to any one of items (1) to (10), wherein enantiomerically enriched compound of Formula *(R)*-III 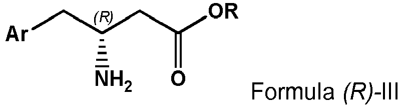 wherein Ar and R are defined as above, is used to subsequently prepare a pharmaceutically active compound.
(13) The process according to item (12), wherein the compound of Formula *(R)*-III, or if R=H the compound of Formula *(R)*-III', is subsequently used to prepare a gliptin compound (DPP-4 inhibitor), in particular to prepare sitagliptin.
(14) The process according to any one of the preceding items, wherein, after the kinetic resolution step (ii), the compound of Formula *(S)*-III in enantiomerically enriched form 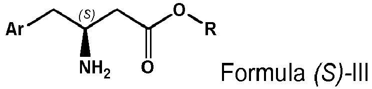 wherein Ar and R are defined as above,
   is oxidized to its C-N double bonded intermediate by means of an oxidizing agent, and the thus formed C-N double bonded intermediate is reduced by means of a reducing agent, in order to obtain the compound of formula III in a racemic form, 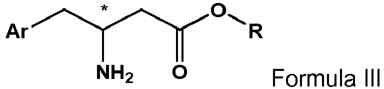 which compound of formula III in a racemic form is recycled in the process by being N-acylated according to step (i) of item (1) in order to eventually prepare the compound of Formula *(R)*-II.
   According to this item, a recycling concept is provided, showing that an undesired enantiomer can be reused. The enantioenriched compound of Formula *(S)*-III gained as a byproduct of bioconversion by penicillin amidase can be converted to its racemate, that is, up to 50% of an "undesired" enantiomer can be converted to the desired enantiomer. The desired R-enantiomer can be reintroduced or provided as starting material in a preparation process for preparing the compound of Formula *(R)*-II, preferably in a further process for finally preparing a gliptin compound (DDP 4-inhibitor), while then the undesired S-enantiomer can be again subjected to the racemisation process.
(15) A process for preparing a gliptin compound (DPP-4 inhibitor) of Formula I or a salt thereof 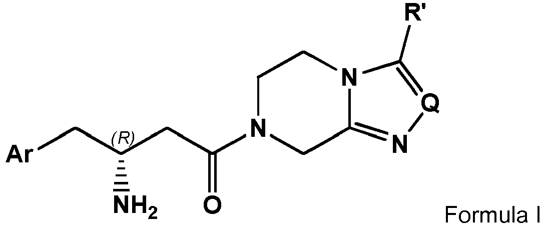 wherein Ar denotes unsubstituted or substituted C₆-C₁₂-aryl, Q denotes N, CH or a carbon substituted with unsubstituted or substituted C₁-C₆-alkyl, C₆-C₁₂-aryl or C₇-C₁₂-alkylaryl, and R' denotes H or unsubstituted or substituted C₁-C₆-alkyl, C₆-C₁₂-aryl or C₇-C₁₂-alkylaryl,
   the process comprising:
   (i) preparing γ-aryl-β-amino carboxylic acid of Formula *(R)-*III' 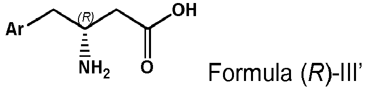 wherein Ar is defined as above,
      involving a biocatalytic reaction using penicillin amidase,
   (ii) cyclodehydration of the compound of Formula *(R)*-III' to obtain a β-lactam of Formula *(R)*-V 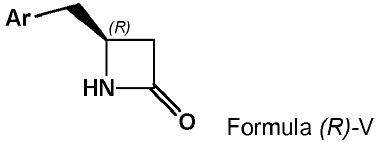 wherein Ar is defined as above,
   (iii) reacting the compound of Formula *(R)*-V with a compound of formula VI or salt thereof, 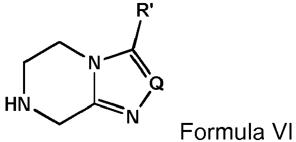 wherein R' and Q are defined as above,
      in the presence of a catalyst in an organic solvent.
(16) The process according to item (15), wherein the γ-aryl-β-amino carboxylic acid of Formula *(R)*-III' is prepared by a process as defined in any one of items (1) to (10), in particular item (10).
(17) The process according to item (15) or (16), wherein the prepared gliptin compound is sitagliptin or a salt thereof.
(18) A compound of formula II 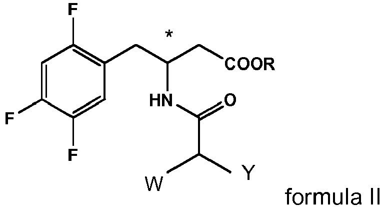
   wherein R is H or C₁-C₄ alkyl,
   W denotes unsubstituted or substituted C₆-C₁₂-aryl, furanyl or thienyl and Y independently denotes hydrogen, hydroxy, amino, chloro, bromo or methyl, or
   W denotes unsubstituted or substituted C₆-C₁₂-aryloxy, C₅-C₁₂-heterocycle or -heteroaryl, and Y is hydrogen, wherein the compound of formula II is in racemic form or is enantiomerically enriched in either (*R*)- or (*S*)-form.

   With respect to the definitions of the term "C₆-C₁₂-aryl" and of the term "C₅-C₁₂-heterocycle or -heteroaryl" it is referred to item (1) above.
(19) The compound according to item (18), wherein W is phenyl and Y is H.
(20) 4-(2,4,5-trifluorophenyl)-3-phenylacetylaminobutyric acid of Formula IIa 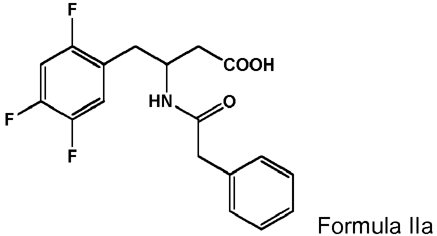
(21) A compound of Formula *(R)*-IIa: 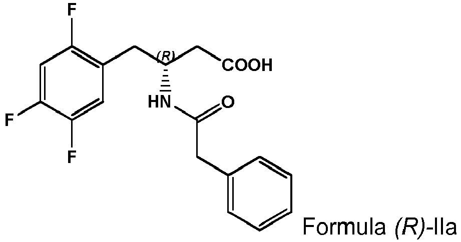
(22) A compound of Formula *(S)*-IIa: 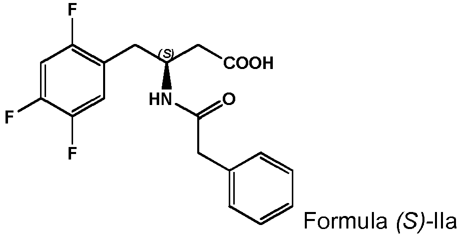
(23) Use of a compound as set forth in any one of items (18) to (22) in a process for preparing a gliptin compound (DPP-4 inhibitor).
(24) A process for preparing a pharmaceutical composition comprising a gliptin compound of Formula I or a pharmaceutically acceptable salt thereof 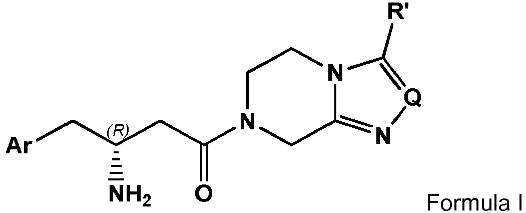 wherein Ar denotes unsubstituted or substituted C₆-C₁₂-aryl, Q denotes N, CH or a carbon substituted with unsubstituted or substituted C₁-C₆-alkyl, C₆-C₁₂-aryl or C₇-C₁₂-alkylaryl, and R' denotes H or unsubstituted or substituted C₁-C₆-alkyl, C₆-C₁₂-aryl or C₇-C₁₂-alkylaryl,
   the process comprising:
   carrying out a process according to any one of items (13) and (15) to (17) for preparing said gliptin compound of Formula I or a pharmaceutically acceptable salt thereof, and
   mixing the prepared gliptin compound of Formula I with at least one pharmaceutically active ingredient to obtain a pharmaceutical composition.
(25) The process according to item (24), wherein said gliptin compound is combined with another pharmaceutically active ingredient, either within the same pharmaceutical composition or another pharmaceutical composition, wherein said another pharmaceutically active ingredient is selected from the group consisting of insulin sensitizers, insulin, insulin mimetics, sulfonylureas, (-glucosidase inhibitors, glucagon receptor antagonists, GLP-1, GLP-1 analogues, GLP-1 mimetics, GLP-1 receptor agonists, GIP, GIP mimetics, PACAP, PACAP mimetics, PACAP receptor agonists, cholesterol lowering agents, PPAR-δ agonists, anti-obesity compounds, ileal bile acid transporter inhibitors, agents intended for use in inflammatory conditions, antihypertensive agents, glucokinase activators (GKAs), inhibitors of 11(-hydroxysteroid dehydrogenase type 1, inhibitors of cholesteryl ester transfer protein (CETP) and inhibitors of fructose 1,6-bisphosphatase.

### Detailed Description of the Invention

The present invention will now be described in further detail with respect to preferred embodiments and specific examples, which embodiments and examples are however presented for illustrative purposes only and shall not be understood as limiting the scope of the present invention.

According to the present invention, a cheap biocatalytic way was found for direct creation of the chiral amino substituted carbon center at the γ-aryl-β-aminobutyric structural moiety using penicillin amidases (penG acylases), which optionally can be chosen from a commercially available enzyme, and which have not been contemplated or suggested or found out for use of preparation of the particular structural characteristic of dipeptidyl peptidase-4 (DPP-4) inhibitors, and sitagliptin especially.

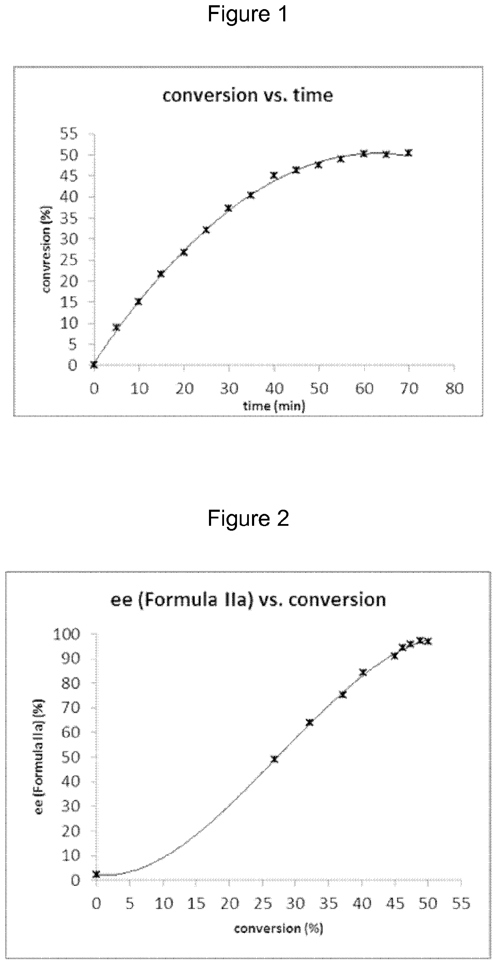

Penicillin amidases (penG acylases) are currently examples of the enzymes most widely applied industrially for the production of semi-synthetic antibiotics, but they are also applied for the synthesis of enantiomerically pure L-amino acids through enantioselective enzymatic hydrolysis of N-phenylacetyl-D,L-amino acids in aqueous solution. So, they represent one of the cheapest and most available biochemical ways for preparation of L-α-amino acids (mostly configuration *(S)* according to Cahn-Ingold-Prelog rule) through cleavage of phenylacetylamide bond. For suitable use in industrial applications, however, the enzymatic resolutions have to follow high enantioselectivity characterised by the E value of the specific resolution system and in addition the activity of the catalyst towards the desired substrate has to be high enough to allow industrialy suitable loads of the biocatalyst and acceptable lengths of the process. The current state of the in art in biocatalytic conversions using penG acylase does not provide a general rule for prediction of reactivity and stereoselectivity of the enzymes toward uncharacterised substrate-enzyme pairs. A good example of high variability in reactivity arising from various structural properties of the substrate (substituents of the basic α-amino acid or β-aminoacid frame) is known in the art (Monatshefte Chem. 131, 623 (2000); in particular Fig.1 thereof) wherein the reaction rates using various substrates can differ by more than 100 fold. It has also been observed that some specific structural properties of amino acids or their esters/amides (such as phenylalanine moiety in dipeptides) to be resolved with penicillin amidase, have a particularly negative role in reactivity of the enzyme (Tetrahedron Lett. 29, 1131 (1988)).

The activity of penicillin amidases on β-amino acids have been studied by Soloshonok (Synlett 339 (1993); Tetrahedron: Asymmetry 6, 1601 (1995)) and a couple of further published references (Tetrahedron: Asymmetry 5, 1119 (1994) and 5, 1225 (1994); J. Org. Chem. 63, 2351 (1998); Eur. J. Org. Chem. 155 (1999); Bioorg. Med. Chem. Lett. 10, 1755 (2000); Tetrahedron Lett. 41, 681 (2000) and 42, 8997 (2001); Biotechnology Lett. 29, 1825 (2007); Shengwu Jiagong Guocheng 8, 13 (2010)). The published works were mostly limited to β-aryl and β-fluoroalkyl substituted β-amino, acids. Furthermore, substrates for preparation of DPP-4 inhibitors have *(R)*-configuration according to Cahn-Ingold-Prelog rule, which is opposite to the product of enzymatic hydrolysis.

From the prior art it was difficult to expect efficient hydrolysis of molecules with Formula II of the invention with penicillin amidasein view of the following knowledge or presumptions: firstly a very large difference in reaction velocities exist for structuraly distinct substrates (Monatshefte Chem., 131, 623 (2000); Fig.1) and secondly the 2-aryl-1-aminoethyl moiety of the molecules of formula II is analogous in part to phenylalanine, which is even known in some cases as an inhibitor of penicillin amidase enzymatic activity (Tetrahedron Lett. 29, 1131 (1988)). In addition to the lack of descriptions and hints of reactivity of N-acyl derivatives of γ-aryl-β-amino acids with penicillin amidase in the prior art, an even more questionable property of such enzyme is the ability to discriminate between the enantiomers of said compounds.
It was therefore surprising to find, that the penicillin amidase catalyzed hydrolysis of N-aryl acetyl- (such as phenyl acetyl-) substituted γ-aryl-β-amino acids proceeds not only with highly desired rates but also in excellent enantioselectivity. The estimated E value for various combinations of the kinetic resolution reaction is above 40, reaching 100 in the preferred embodiment of the invention.
Hence, according to the unexpected findings and representative for the compounds of formula II used according to the present invention, the (R)-isomer of compound of Formula IIa is hydrolysed much slower, which is observed from experimentally determined progress curve of enantiomeric purity of compound of Formula (*R*)-IIa. Herein it was shown that enantiomeric purity of compound of Formula (*R*)-IIa increases with the progress of conversion, reaching plateau with ee of 99.5% at approximately 51% of conversion (Figure 1). It could be also seen that after reaching value of approximately 50% the conversion practically stops (Figure 2).
This is highly in favour of the intention for preparation of *(R)*-isomer, as a valuable starting material for preparation of gliptins (DPP-4 inhibitors).

Thus, the present invention surprisingly satisfies the hitherto unmet need for a biocatalytic preparation, using cheap and readily available enzyme, of substrates for preparation of enantiopure pharmaceutically active agents respectively having a structural moiety defined by γ-aryl-β-aminobutyric acids and their derivatives such as acid amides and acid esters, and variants of the compounds differing in aryl and/or amine substituents, in particular gliptins (DPP-4 inhibitors) and especially sitagliptin.

According to preferred embodiment, the present invention provides an enantioselective process for the preparation of an intermediate of Formula *(R)*-II: 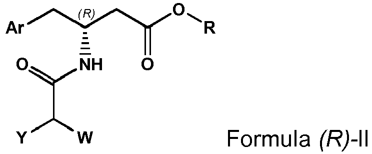 wherein
Ar is unsubstituted or substituted C₆-C₁₂-aryl, R is C₁-C₄ alkyl, and
W denotes unsubstituted or substituted C₆-C₁₂-aryl, furanyl or thienyl and Y independently denotes hydrogen, hydroxy, amino, chloro, bromo or methyl, or
W denotes unsubstituted or substituted C₆-C₁₂-aryloxy, C₅-C₁₂-heterocycle or -heteroaryl, and Y is hydrogen,
   comprising
   (i) N-acylation of racemic γ-aryl-β-aminobutyric acid derivative of Formula-III, 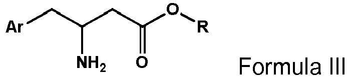 or a mixture of enantiomers with poor excess of one enantiomer, wherein Ar is defined as above
      with an activated acid derivative of the compound of Formula IV 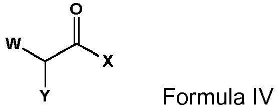 wherein Y and W are defined as above and X is a leaving group in a solvent and a presence of a base to afford racemic substituted acetylated γ-aryl-β-aminobutyric acid derivative of Formula II 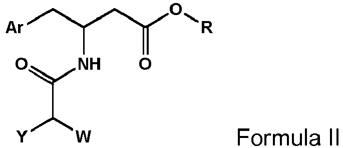
   (ii) kinetic resolution of racemic derivative of Formula II with penicillin amidase enzyme in an aqueous solvent to a highly enantiomerically enriched compound of Formula *(R)*-II and enantiomerically enriched compound of Formula *(S)*-III 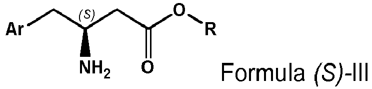 wherein Ar is defined as above
   (iii) obtaining the compound of Formula *(R)*-II, preferably by extraction thereof from the aqueous medium, which is further preferably rendered acidic, with a water immiscible solvent
   (iv) optionally removing solvent.

The term "C₆-C₁₂-aryl" is as defined above, preferably represents phenyl, 1-naphtyl, or 2-naphtyl.
The term "C₅-C₁₂-heterocycle or -heteroaryl" is as defined above, preferably represents 1-tetrazolyl, oxazolyl, phenoxazolyl, 2-oxo-3-oxazolyl and 4,5-phen-2-oxo-3-oxazolyl
Ar is more preferably selected from substituted phenyl, in particular halo substituted phenyl, still more preferably fluoro substituted phenyl, most preferably 2,4,5-trifluorophenyl.
X is most preferably chloro.
W is most preferably phenyl, and Y, R are most preferably H.

According to a further preferred embodiment the present invention relates to an enantioselective process for the preparation of an intermediate of Formula *(R)*-IIa: 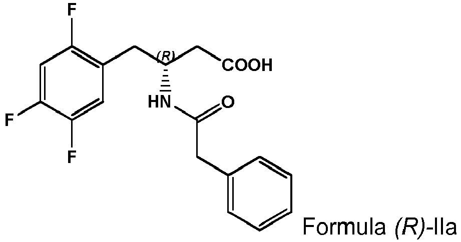 comprising
(i) N-acylation of racemic 4-(2,4,5-trifluorophenyl)-3-aminobutyric acid of Formula IIIa, 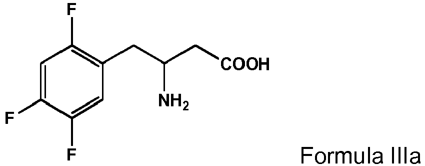 or a mixture of enantiomers with poor excess of one enantiomer, with an activated derivative of phenylacetic acid preferably phenylacetyl chloride in a solvent and a presence of a base to afford racemic 4-(2,4,5-trifluorophenyl)-3-phenylacetylaminobutyric acid of Formula IIa 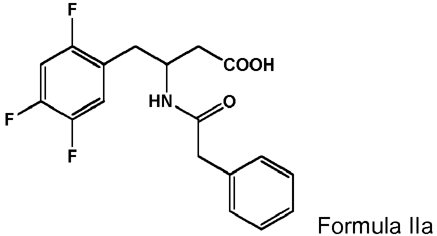
(ii) kinetic resolution of racemic derivative of Formula IIa with penicillin amidase enzyme in an aqueous solvent until highly enantiomerically enriched compound of Formula *(R)*-IIa and enantiomerically enriched compound of Formula (*S*)-IIIa are obtained. 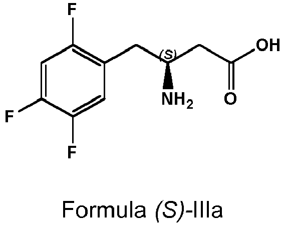
(iii) obtaining the compound of Formula *(R)*-II, preferably by extraction thereof from acidic aqueous medium with a water immiscible solvent
(iv) optionally removing solvent.

The acylation with activated aryl-(preferably phenyl-)acetic acid derivative, preferably phenylacetyl chloride is carried out in a solvent, which can be suitably selected from chlorinated hydrocarbons such as methylene chloride, nitriles such as acetonitrile, amides such as N,N-dimethylformamide or N,N-dimethylacetamide, ketones such as acetone, esters such as ethyl acetate, cyclic ethers such as tetrahydrofuran, and water. The most preferred solvent is acetone - water mixture. The acylation is carried out in the presence of a base, which preferably is selected from hydroxides, inorganic carbonates or organic amines, preferably triethylamine or diisopropylethylamine. The most preferred solvent/base medium is a mixture of triethylamine, acetone and water. Preferably, the activated phenylacetic acid derivative is added dropwise at a lower temperature, preferably from -5 to 5 °C, afterwards the reaction can be carried out at higher temperature, such as from 20 to 40 °C, preferably at room temperature.

The term penicillin amidase or the alternative terms penicillin acylase, penicillin amidohydrolase, penicillin G acylase, penicillin G amidohydrolase, penicillin V acylase and penicillin V amidase comprises enzyme homologs from various organisms under common enzyme classification number EC 3.5.1.11 and are well-known in the β-lactam art as enzymes that catalyze the hydrolysis of the N-acyl side chain from penicillins and cehalosporins. It is commonly used for removal of penicillin G, penicillin V and amoxycillin N-acyl side chain phenylacetyl, phenoxyacetyl and phenylglycyl side chain, respectively (Org. Proc. Research & Dev. 2, 128 (1998)), however substrates wherein Y is OH, Cl, Br, Me are also well accepted by penicillin acylase (Bioorg Med Chem Lett, 4, 345 (1994)). The acetyl groups with other substituents, such as C₆-C₁₂-aryloxy or 1-tetrazolyl (Tetrahedron Lett, 38, 4693 (1997)) or 3-thienyl (Bioorg Med Chem Lett, 4, 345 (1994) are also found to be good substrates for penicillin amidases in the synthesis of cefalosporins. Chiral resolution of some β-amino acid esters is also described, wherein also longer aliphatic N-acyl side chains of β-lactams could be removed (Bioorg & Med. Chem, 7, 2221(1999)).

Beside hydrolysis, N-acyl transferase activity of penicillin amidase is used for N-acylation of 6-aminopenicillanic acid and 7-aminodeacetoxycephalosporanic acid. Beside β-lactams many other subtrates are known, for example, α-, β-, and γ-amino acids, aminophosphonic acids, peptides, alkyl or aromatic amines and hydrazides. Penicillin amidases from various biological sources display various specificities towards their substrates (FEBS Lett 1997, Vol. 417; p.414). Penicillin amidases suitable for use in the process of the present invention found by known methodology from many organisms and used as wild-type or mutant form, for example, from Gammaproteobacteria, *Escherichia coli, Bacillus sp., Bacillus megaterium, Bacillus badius, Bacillus subtilis, Achromobacter sp., Achromobacter xylosoxidans, Acetobacter sp., Xanthomonas sp., Pseudomonas sp., Pseudomonas melanogenum, Gluconobacter suboxydans, Microbacterium dimorpha, Proteus alboflavus, Kluyvera citrophila, Kluyvera cryocrescens, Providencia rettgeri, Actinoplanes sp., Actinoplanes utahensis, Alcaligenes faecalis, Arthrobacter viscosus, Lactobacillus plantarum, Lysinibacillus sphaericus, Erwinia aroideae, Rhodotorula aurantiaca, Fusarium sp., Thermus thermophilus, Streptomyces sp., Streptomyces mobaraensis, Streptomyces lavendulae, Penicillium chrysogenum, Mucor griseocyanus* and from any other organism, which is found to express penicillin amidase activity. Penicillin amidase found in *E. coli, P. rettgeri, B. megaterium, Achromobacter sp.* or *Alcaligenes faecalis* is preferred. Penicillin G amidase found in *E.coli* is mostly preferred.

The enzyme chosen from the penicillin amidases or penicillin acylases may be comprised within a living whole cell, or is comprised within an inactivated whole cell or is comprised within a homogenized whole cell, or is comprised within an immobilized whole cell, or is comprised within a cell free extract or is an extracellulary expressed protein or is a partially or substantially purified enzyme.

Once purified, the penicillin amidase may be used in a "free" form, i.e., solubilized liophylisate in aqueous or substantially aqueous solutions, or may be immobilized onto a support matrix such as an intermolecular adduct with glutaraldehyde; Sepharose™; Sephadex G-200™, acrylamide, N,N-methylenebis(acrylamide) and maleic anhydride; Dextran™; maleic anhydride; tetramethylene glycol; dimethacrylate; methacrylic acid, DEAE-Cellulose™; CM-Cellulose™; AE-Cellulose™; and other cellulose derivatives; CM-Sephadex Amberlite IRC-50™ and other weak cation and anion exchangers; ethylene maleic anhydride copolymers; Nylon™; Amberlite XAD-7™; sucrose / epichlorohydrin copolymer; polyacrylamide; cellulose; intermolecular adduct with glutaraldehyde; acrylamide copolymer; anion exchange phenolformaldehyde resin; DEAE-Sephadex™; glycidyl methacrylate; methylene bisacrylamide; diatomaceous earth; silica gel, poly(hydroxyethylmethacrylate); Eupergit C™; basic anion exchanger (polyamine); styrene; divinyl benzene; cellulose triacetate fibres; AH-Sepharose™ / benzoquinone; nitrocellulose fibres; a polyethylene imine; Bentonite(TM); a polyacrylamide gel entrapment or derivatised polyacrylonitrile, gelatin, alginate amine, chitosan, pectin and any other type of support matrix known in the present state of the art. Both, immobilized and liophylized penicillin amidase may be obtained commercially.

Therefore the present invention can be carried out using any form of penicillin amidase/penicillin acylase enzyme, whether used within a living whole cell, within an inactivated whole cell, within a homogenized whole cell, within an immobilized whole cell, within a cell free extract, in an extracellulary expressed form, in a free form or immobilized on a support matrix. Preferably, however, the enzyme used is immobilized on a solid support matrix, because such catalysts can be reused several times due to convenient separation from other reaction components.

The amount of penicillin amidase/penicillin acylase present in the reaction mixture as well as specific reactivity of a particular enzyme toward the desired substrate dictates the rate of the reaction. The amount of enzyme in the reaction is expressed in units of enzymatic activity. In the context used herein, one unit (U) is the amount of enzyme that will catalyse hydrolysis of one micromole of penicillin G in one minute at 28°C.

As it is evident from the examples presented, the time and yield of kinetic resolution depends on the amount of the enzyme in the reaction. If the amount of the enzyme with respect to the substrate in the reaction is too high this could lead to shorter reaction times, which are however hard to manipulate on an industrial level and could result in a lower yield and enantiomeric purity of the recovered enantiomer. If insufficient amount of the enzyme with respect to the substrate is added, the reaction will be slower and may be industrially less useful.

For purpose of illustration, if the intrinsic reactivity of the enzyme-substrate pair is low, the amount of the enzyme needed to achieve industrially suitable reaction times would be very high, influencing process costs and volumetric productivity of the process. In light of extremely high effect of the substrate structure to reaction velocity described in the art, we were surprised to find that the reactivity of penicillin amidase/penicillin acylase from several organisms, in particular penicillin G amidase, either in free or immobilised form allow sufficient reaction rates with compound of Formula II and enantioselectivity (observed in substantially faster formation of compound of Formula *(S)*-III compared to the opposite enantiomer) for an efficient, industrially scalable and cost effective process.

Preferably, 5 - 2000 U of penicillin amidase per 1 g of substrate is used. More preferably 20 - 1500 U of penicillin amidase per 1 g of substrate is used. The most preferably 50-1000 U of enzyme per 1 g of substrate is used.

For the kinetic resolution of the present invention water is the preferred medium. The aqueous system may be supplemented with various inorganic or organic acids or bases for purpose of pH correction, buffering, enzyme activity and stability enhancement as well as solubility enhancers such as surfactants and detergents.

In addition the reaction may be carried out in an aqueous medium supplemented with various polar organic solvents comprising from 1 to 28% of a polar organic solvent such as acetone, tetrahydrofuran, methanol, ethanol, 1-propanol, 2-propanol, acetonitrile, dimethylsulfoxide, propylene glycol methyl ether, propylene glycol, ethylene glycol, dimethyl ether, 2-methoxyethyl ether, ethylene glycol, or glycerol, and from 99% to 72% water.

Biphasic mixtures of water or aqueous medium with water insoluble organic solvents may also be used. The said water insoluble organic solvent may be selected from esters, such as methyl acetate, ethyl acetate, propyl acetate, nonpolar ethers such as diethyl ether, diisopropyl ether, methyl tert-butyl ether, chlorinated solvents such as dichloromethane, chloroform and from aromatic solvents such as toluene, chlorobenzene etc.

In a preferred embodiment, the kinetic resolution of the present invention may be carried out in aqueous media at a pH of 3 to 11, more preferably at a pH of 6 to 9 and most preferably at a pH of 6.5 to 8.5.

The temperature at which the process may be carried out will be appreciated by one of ordinary skills in enzyme catalysis and thus is not a critical limitation of the process providing the temperature does not exceed the deactivation temperature for the used enzyme; however, a temperature range of 5°C to 65°C is preferred. The temperature range of 20°C to 40°C is more preferred. The most highly preferred temperature is 25°C to 35°C.

In further step of the process of the invention the enzyme is removed appropriately, depending on the form of the catalyst used. Well known methods for removal of proteins comprised either within a living whole cell, within an inactivated whole cell, within a homogenized whole cell, within an immobilized whole cell, within a cell free extract, in an extracellulary expressed form, in free form or immobilized on a support matrix are known in the art. In a preferred embodiment, the use of enzyme immobilized on a support matrix simplifies the removal by application of simple sieving, filtration or other straightforward solid/liquid separation techniques.

Next, the product of the bioreaction is obtained. This is suitably carried out by separation from the non-transformed isomer by acidifying the aqueous medium with an acid, preferably a strong acid and more preferably selected from inorganic acid such as hydrochloric or sulphuric acid, to adjust the pH value below 3, preferably below 2. The protonated unprotected amino acid remains in the aqueous medium, while the protected compound is extracted with a water immiscible solvent selected from esters, chlorinated hydrocarbons, ethers or aromatic hydrocarbons, preferably from esters such as ethyl acetate or isopropyl acetate.

The protected product of Formula *(R)*-II, *(R)*-II' or *(R)*-IIa can be used directly for further reaction steps, or can be isolated, for example by evaporation of the solvent, optionally after drying with solid inorganic drying agent. Optionally the product is transferred to next steps by partial or complete retention of the solvent.

The deprotected enantiomer of Formula *(S)*-III or *(S)*-IIIa can be isolated from the aqueous medium, suitable by alkalising to a pH value of 6-8, preferably 7, and by filtration, optionally by removing ionic component by conventional methods such as reverse osmosis, ion exchange resin adsorption or ion exchange chromatography, followed by evaporation of the solvent.

According to further aspect the present invention relates to an enantioselective process for the preparation of an intermediate of Formula *(R)*-III': 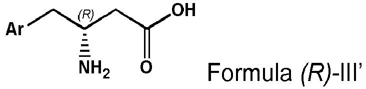 wherein Ar denote unsubstituted or substituted C₆-C₁₂-aryl, comprising
(i) N-acylation of racemic γ-aryl-β-aminobutyric acid derivative of Formula III', 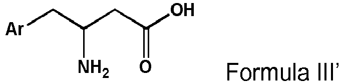 or a mixture of enantiomers with poor excess of one enantiomer, wherein Ar is defined as above,
   with an activated acid derivative of the compound of Formula IV 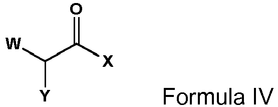 wherein Y and W are defined as above and X is a leaving group, in a solvent and in the presence of a base to afford racemic substituted acetylated γ-aryl-β-aminobutyric acid derivative of Formula II' 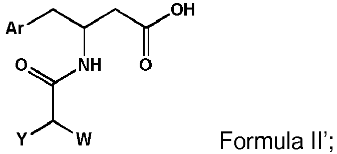
(ii) kinetic resolution of racemic derivative of Formula II' with penicillin amidase enzyme in an aqueous solvent to a highly enantiomerically enriched compound of Formula *(R)-*II' 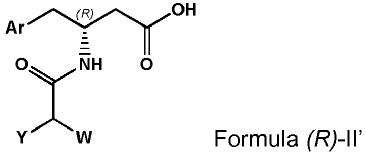 wherein Ar, Y and W are defined as above;
(iii) obtaining the compound of Formula *(R)*-II', preferably by extraction thereof from acidic aqueous medium with a water immiscible solvent followed by complete or partial removal of the solvent; and
(iv) hydrolysing the compound of Formula *(R)*-II' to the compound of Formula *(R)*-III'.

According to a preferred option of this aspect, Ar is 2,4,5-trifluorophenyl, Y is H and W is phenyl, and the compound of Formula *(R)*-IIIa: 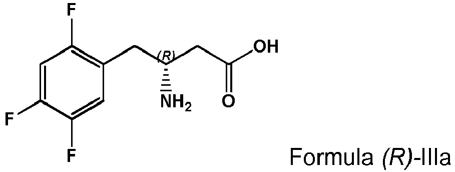 is prepared following the same process using starting materials and intermediates containing corresponding substituents.

Hydrolysis of the compounds of Formulae *(R)*-II, *(R)-*II' or (*R*)-IIa can suitably be carried out in a protic solvent, preferably one selected from alcohols and water or a mixture thereof, using a strong acid, preferably selected from strong inorganic acids such as hydrochloric acid or sulphuric acid. Most preferably the hydrolysis is performed in aqueous solution of hydrochloric acid of concentration at least 2M, preferably at least 6M. The reaction is preferably carried out at higher temperature, more preferably at reflux till completeness, preferably at least 2 hours, most preferably at least 5 hours.

In one further embodiment of the invention the compound of Formula *(R)*-III is used for preparation of gliptins (DPP-4 inhibitors). A skilled person may follow known synthetic schemes such as the preparation in Scheme 4 or other reaction schemes known or apparent to the skilled person. For example, the compound of Formula *(R)*-IIIa is *de facto* an enantiomer of the compound (±)-**21**, which can be further reprotected, coupled with a heterocycle and deprotected to give sitagliptin. A requirement for reprotection/deprotection scenario unfavourably prolongs the preparation of DPP-4 inhibitors. In order to take advantages of the present invention a shorter process should be performed.

Thus, according to a preferred aspect of preparation of gliptins or DPP-4 inhibitors, the compound of Formula I 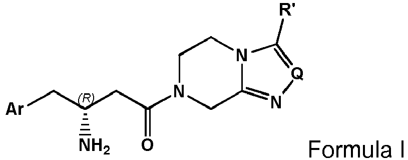 wherein Ar denotes unsubstituted or substituted C₆-C₁₂-aryl, Q denotes N, CH or a carbon substituted with unsubstituted or substituted C₁-C₆-alkyl, C₆-C₁₂-aryl or C₇-C₁₂-alkylaryl, and R' denotes H or unsubstituted or substituted C₁-C₆-alkyl, C₆-C₁₂-aryl or C₇-C₁₂-alkylaryl,
is prepared by a process, comprising
(i) preparation of γ-aryl-β-amino carboxylic acid of Formula *(R)*-III 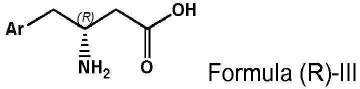 wherein Ar is defined as above,
   by a biocatalytic reaction using penicillin amidase,
(ii) cyclodehydration of the compound of Formula *(R)*-III to obtain a β-lactam of Formula *(R)*-V 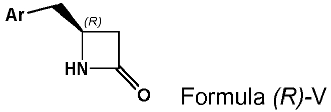 wherein Ar is defined as above,
(iii) reacting the compound of Formula *(R)-V* with a compound of formula VI or salt thereof, 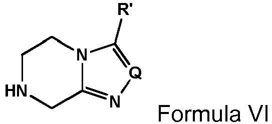 wherein R' and Q are defined as above,
   in the presence of a catalyst in an organic solvent.

According to a preferred embodiment of this aspect of the invention, compound(s) of formula I, *(R)*-III, *(R)*-V and/or VI is/are characterized by either one or a combination of the following features a) to e):
a) in compounds of formulae I, *(R)*-III and *(R)*-V, Ar is substituted phenyl, preferably halo substituted phenyl, more preferably fluorine substituted phenyl, in particular 2,4,5-trifluorophenyl;
b) in compounds of formula I and VI, R' is substituted C₁-C₃-alkyl, preferably halogen substituted C₁-C₃-alkyl, more preferably fluoro substituted C₁-C₃-alkyl, in particular trifluoromethyl;
c) in compounds of formula I and VI, Q is N;
d) Ar is 2,4,5-trifluorophenyl, R' is trifluoromethyl, Q is N
e) compound of formula I is provided in the form of its pharmaceutically acceptable acid addition salt, preferably in the form of its phosphate salt,

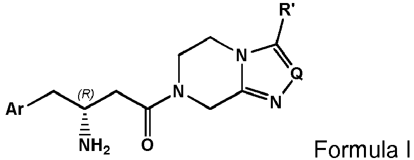

According to a special item d) of this aspect of the invention, the final product is sitagliptin (Formula la)

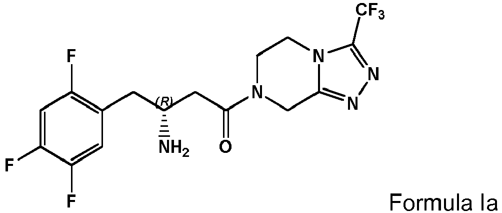

Preferably, cyclodehydration of γ-aryl-β-amino carboxylic acid of Formula *(R)*-III to β**-**lactams is carried out by applying various dehydration reagents such as carbodiimides, preferably dicyclohexylcarbodiimide, triphenylphosphine in combination with tetrahalomethanes, with N-bromo compounds, such as N-bromosuccinimide, or with disulfides or by transforming the acid to highly reactive derivatives such as acid chlorides or mixed anhydrides with phosphonic or sulfonic acids. More preferably cyclodehydration of β-amino carboxylic acids to β-lactams is carried out by applying a C₁-C₆-alkylsulfonyl chloride in combination with a proton acceptor which converted the mixed anhydride to β-lactams. Preferably methanesulfonyl chloride (MeSO₂Cl) and NaHCO₃ are applied as reactants. Thereby, a combination of reagents is selected which provides for particularly effective cyclodehydration reaction.

According to a preferred embodiment, compound of Formula *(R)*-III applied as the starting material for cyclodehydration reaction is prepared according to the enzymatic kinetic resolution process using penicillin amidase described above.

In a preferred option of item (iii), the coupling process is characterized by either one or a combination of the following procedural features:
- the solvent is selected from the group of C₂-C₈ aliphatic ethers, C₄-C₆ cyclic ethers, C₁-C₄-alkyl C₁-C₄-alkanoates and C₁-C₄-alcohols, preferably the solvent is selected from symmetric di-(C₂-C₄-alkyl) ethers or asymmetric di-(C₁-C₄-alkyl) ethers, tetrahydrofuran, methanol, toluene, hexane or isopropyl acetate, in particular the solvent is tetrahydrofuran; and/or
- the catalyst is selected from organic and inorganic proton donators, preferably the catalyst is selected from H₂O, HCl or C₁-C₁₂-alkanecarboxylic acid, preferably C₄-C₁₀-alkanecarboxylic acid, more preferably C₇-C₉-alkanecarboxylic acid, in particular 2-ethylhexanoic acid (2-EHA).

Thereby, solvent and catalyst are suitably selected in order to provide for particularly advantageous conversion rate to compound of formula V.

According to a further aspect of the invention, a recycling concept is provided in order to reuse the undesired enantiomer. In particular, enantioenriched compound of Formula *(S)*-III gained as a byproduct of bioconversion by penicillin amidase can be converted to its racemate, that is, up to 50% of an "undesired" enantiomer can be converted to the desired enantiomer. The desired R-enantiomer can be provided as starting material in a preparation process, preferably a process for preparation of gliptins/DDP 4-inhibitors, while the undesired S-enantiomer can be again subjected to the racemisation process.

According to this aspect of the invention, a process for preparing compound of Formula III' 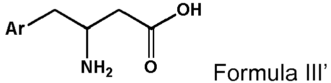 may preferably comprise the steps of:
(a) oxidation of a compound of Formula *(S)*-III in enantiomerically enriched form to its C-N double bonded intermediates by means of an oxidizing agent, and
(b) reduction of the C-N double bonded intermediates obtained in step i) by means of a reducing agent,
in order to obtain compound of formula III' as a racemic form.

Preferably, both steps (a) and (b) of the racemisation process are carried out in a one-pot process. That is, steps (a) and (b) are carried out in the same reaction vessel. In a preferred embodiment the same solvents are used in both steps. Alternatively, solvents used in the oxidation step are easily removed and replaced by the solvent suitable for the reduction step. Preferably, oxidizing and reducing agents are specially selected to act in media, which allow one-pot procedure.

According to a further preferred embodiment of this aspect of the invention, oxidation agents are selected to efficiently racemise primary amines *via* C-N double bonded intermediates. Preferably, oximes as a tautomeric form of nitroso intermediates are the sufficiently stable C-N double bonded representatives, used for such conversion.

Preferably, oxidation agents are selected from tungsten (VI) compounds, preferably Na₂WO₄ or WO₃ in molar amounts or catalytic amounts with co-oxidant such as hydrogen peroxide, various peroxo compounds, formed in situ from hydrogen peroxide and a compound which is transformed to an active peroxo intermediate and is selected tungsten, vanadium, molybdenum compounds or used as isolated reagents such as dioxiranes, such as dimethyldioxirane, perbenzoic acids and salts, peroxysulfuric acid and salts. The most preferred reagents are sodium (VI) tungstate, dimethyldioxirane and potassium peroxymonosulfate (KHSO₅), particularly in the commercial product Oxone®. Oxone is a complex mixture of derivatives of sulfuric acid in which potassium peroxymonosulfate is the active reagent. It is preferably used in acetone, in which dimethyloxirane is formed *in situ* as an active oxidizing reagent.

Preferably, reducing methods for conversion of N-oxyamino and/or N-oxyimino intermediates to racemic primary amines are selected from catalytic hydrogenation on palladium catalyst on a supporter as a preferred catalyst or from reduction with metals form low oxidation states, such as Fe²⁺, Sn²⁺, elemental tin and zinc, most preferably elemental zinc in acidic medium is used.

According to a particularly preferred embodiment, the oxidizing agent is potassium peroxymonosulfate (KHSO₅) and/or the reducing agent is elemental zinc (Zn). Thereby, a particularly effective combination of oxidizing agent and reducing agent is selected. Although according to literature (J. Org Chem. **57,** 6759 (1992)) potassium peroxymonosulfate in acetone may lead to a complex mixture of oxidation intermediates, it was surprisingly found that following by reduction with zinc in hydrochloric acid the intermediates are transformed to the unique compound of Formula *rac*-III with high selectivity.

According to another preferred embodiment of this aspect of the invention, in step (a) and (b), pH of the reaction mixtures is suitably adjusted in view of the oxidizing and reducing agent respectively applied. Thus, using potassium peroxymonosulfate in acetone the pH value is adjusted to mild alkaline from 7 to 10, preferably 7 to 9, most preferably pH is 9 in the part of the reaction time. The preferred reagent for pH adjustment is the phosphate buffer and potassium hydroxide for alkalizing. The reduction with elemental zinc is carried out in highly acidic medium with pH lower than 4, preferably lower than 2, accomplished by preceding addition of mineral acid, preferably hydrochloric acid or acetic acid.

According to still another preferred embodiment of this aspect of the invention, subsequent to step (a) and prior to step (b), water and/or organic solvents are removed from the reaction mixture.

According to a particularly preferred embodiment, the oxidizing agent is potassium peroxymonosulfate (KHSO₅) and/or the reducing agent is elemental zinc (Zn).

According to another particularly preferred embodiment, compound of formula Formula III, III' or IIIa is prepared via the above described enzymatic kinetic resolution process. In this way, a particularly desirable recycling concept is provided, since "undesired" enantiomer of compound of Formula III (or III' or IIIa) can be converted to the "desired" enantiomer of compound of Formula III (III' or IIIa). That is, in a reaction affording the enantiomer which is undesired, for example in the above described enzymatic kinetic resolution process; the resulting undesired enantiomer can be recycled by converting it to the desired enantiomer. In particular, in said enzymatic kinetic resolution process, it is useful that at least a part of the amount of compound of Formula III (or III' or IIIa) is prepared according to the aforementioned racemisation process.

Illustrative examples of the overall process of the present invention can be shown for the case of the preparation of sitagliptin, using - without being limited thereto - intermediates wherein Ar is trifluorophenyl, W is phenyl, Y is hydrogen, R' is trifluorophenyl, Q is N, and penicillin amidase is selected from various sources as depicted by examples below.
Such illustrative overall process is shown in the Scheme 6 and described in the examples which follow.

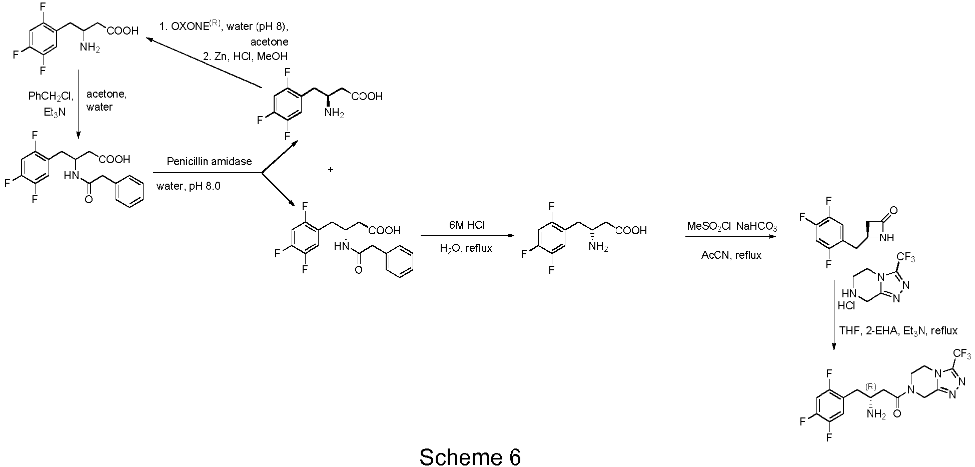

According to another aspect of the invention, a pharmaceutical composition comprising a gliptin compound of Formula I or a pharmaceutically acceptable salt thereof is provided, wherein said gliptin compound is prepared by the process according to the invention as described above and then mixed with at least one pharmaceutically acceptable excipient or carrier to obtain said pharmaceutical composition. As the "pharmaceutically acceptable salt", any typical salt can be used, preferably the gliptin compound and especially sitagliptin is in the form of its phosphate salt. The term "pharmaceutically acceptable excipient" as used herein means any physiologically inert, pharmacologically inactive material known in the art being compatible with the physical and chemical characteristics of the active agent. Preferably, the pharmaceutically acceptable excipient is selected from the group consisting of binders, disintegrants, bulk polymers and preservatives.

According to another preferred embodiment, the pharmaceutical composition comprises at least one additional pharmaceutically active ingredient besides of compound of formula IV, wherein said additional pharmaceutically active ingredient is selected from the group consisting of insulin sensitizers, insulin, insulin mimetics, sulfonylureas, (glucosidase inhibitors, glucagon receptor antagonists, GLP-1, GLP-1 analogues, GLP-1 mimetics, GLP-1 receptor agonists, GIP, GIP mimetics, PACAP, PACAP mimetics, PACAP receptor agonists, cholesterol lowering agents, PPAR-δ agonists, anti-obesity compounds, ileal bile acid transporter inhibitors, agents intended for use in inflammatory conditions, antihypertensive agents, glucokinase activators (GKAs), inhibitors of 11 (-hydroxysteroid dehydrogenase type 1, inhibitors of cholesteryl ester transfer protein (CETP) and inhibitors of fructose 1,6-bisphosphatase. Any such additional pharmaceutically active ingredient other than the gliptin compound is known to the skilled person in the field of controlling blood glucose values (e.g. known from the treatment of diabetes mellitus type 2) and can be used. Preferably, the additional pharmaceutically active ingredient is metformin and/or its pharmaceutically acceptable salt. The gliptin compound obtained by the process according to the present invention can be combined with said other pharmaceutically active ingredient either within the same pharmaceutical composition, or by providing separate pharmaceutical compositions respectively containing gliptin and the another active ingredient.

The following examples are merely illustrative of the present invention and they should not be considered as limiting the scope of the invention in any way. The examples and modifications or other equivalents thereof will become apparent to those versed in the art in the light of the present entire disclosure.

### EXAMPLES

### Example 1: 3-(2-Phenylacetamido)-4-(2,4,5-trifluorophenyl)butanoic acid (IIa)

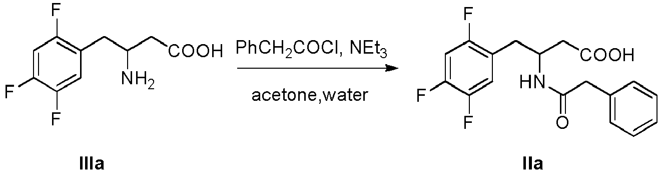

To a solution of racemic 3-amino-4-(2,4,5-trifluorophenyl)butanoic acid (1g; 0.0043 mol) and triethylamine (1.04 g; 0.013 mol) in acetone/water (1:3; 20 mL), phenylacetyl chloride (0.862 g; 0.0056 mol) in acetone (5 mL) is added dropwise over 10 min at 0°C. After 16 h of stirring at ambient temperature, the mixture is filtered and acetone is evaporated. The residue is washed with ether (2 x 30 mL), water phase acidified to pH 1 and extracted with EtOAc (2 x 40 mL). Combined organic phases are dried and concentrated to 1.15 g of white residue, which is recrystallized from EtOAc/Hexane to afford crystalline product.

'H-NMR (500 mHz, DMSO-d6) δ: 2.39 (dd, *J*₁= 7.3 Hz, *J*₂ = 15.6 Hz, 1 H), 2.44 (dd, *J*₁ = 6.4 Hz, *J*₂ = 15.6 Hz, 1 H), 2.65 (dd, *J*₁= 13.8 Hz, *J*₂ = 9,2 Hz, 1 H), 2.82 (dd, *J*₁= 13.8 Hz, *J*₂ = 5.0 Hz, 1 H), 3.30 (2 x d, *J*₁= 14.2 Hz, *J*₂ = 3.3 Hz, 2H), 4.29 (m, 1 H), 7.06 - 7.42 (m, 7H), 8.03 (d, *J* = 8.7 Hz, 1 H), 12.25 (bs, 1 H) ppm).

### Example 2: (R)-3-(2-Phenylacetamido)-4-(2,4,5-trifluorophenyl)butanoic acid ((R)-IIa) and (S)-3-amino-4-(2,4,5-trifluorophenyl)butanoic acid ((S)-IIIa)

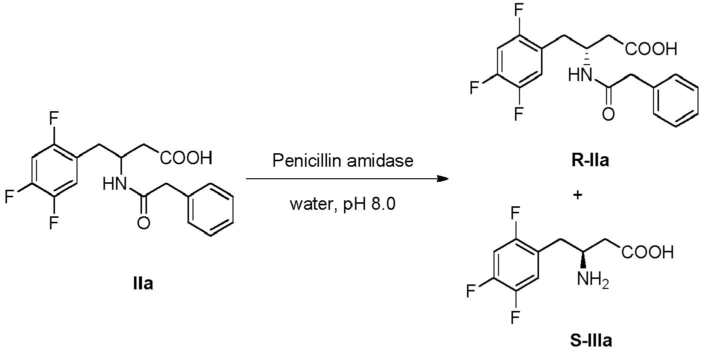

**IIa** (1.0 g; 0.00285 mol) and NaOH (0.003 mol) are dissolved in 30 mL of water and pH of the solution is adjusted to 8.0. The immobilized *E. coli* penicillin amidase (1 g, 250 U) is added and the reaction mixture is stirred at 25°C for 80 min (conversion 42 % determined by HPLC). The enzyme is filtered off, the filtrate is acidified to pH 2 and extracted twice with ethyl acetate. The combined organic phases are washed again with water (pH 2), dried and evaporated to afford 0.59 g of white residue, which contains 0,46 g of highly enantiomerically enriched (ee 94%) **(R)-IIa** and 0.12 g of phenylacetic acid. The residue was processed further without additional purification. The water phase is neutralized to pH 7, concentrated to half volume and cooled to 4°C. The resulting precipitate is filtered off and dried in an oven to afford 0.21 g of enantiomerically enriched **(S)-IIIa** (ee 84%).

### Example 3: (R)-3-(2-Phenylacetamido)-4-(2,4,5-trifluorophenyl)butanoic acid ((R)-IIa) and (S)-3-amino-4-(2,4,5-trifluorophenyl)butanoic acid ((S)-IIIa)

**IIa** (1.0 g; 0.00285 mol) and NaOH (0,003 mol) are dissolved in 30 mL of water and pH of the solution is adjusted to 8.0. The immobilized *E. coli* penicillin amidase (3.5 g, 875 U) is added and the reaction mixture is stirred at 25°C for 70 min (conversion 51 % determined by HPLC). The enzyme is filtered off, the filtrate is acidified to pH 2 and extracted twice with ethyl acetate. The combined organic phases are washed again with water (pH 2), dried and evaporated to afford 0.6 g of white residue, which contains 0.47 g of highly enantiomerically enriched (ee 99.5%) **(R)-IIa** and 0.13 g of phenylacetic acid. The residue was processed further without additional purification. The water phase is neutralized to pH 7, concentrated to half volume and cooled to 4°C. The resulting precipitate is filtered off and dried in an oven to afford 0.27 g of enantiomerically enriched **(S)-IIIa** (ee 80%).

### Example 4: (R)-3-(2-Phenylacetamido)-4-(2,4,5-trifluorophenyl)butanoic acid ((R)-IIa) and (S)-3-amino-4-(2,4,5-trifluorophenyl)butanoic acid ((S)-IIIa)

**IIa** (1.0 g; 0,00285 mol) and NaOH (0,003 mol) are dissolved in 30 mL of water and pH of the solution is adjusted to 8,0. The immobilized *E. coli* penicillin amidase (7 g, 1750 U) is added and the reaction mixture is stirred at 25°C for 35 min (conversion 51 % determined by HPLC). The enzyme is filtered off, the filtrate is acidified to pH 2 and extracted twice with ethyl acetate. The combined organic phases are washed again with water (pH 2), dried and evaporated to afford 0.58 g of white residue, which contains 0.44 g of highly enantiomerically enriched (ee 99,5%) **(R)-IIa** and 0.14 g of phenylacetic acid. The residue was processed further without additional purification. The water phase is neutralized to pH 7, concentrated to half volume and cooled to 4°C. The resulting precipitate is filtered off and dried in an oven to afford 0.25 g of enantiomerically enriched **(S)-IIIa** (ee 78%).

### Example 5: (R)- 3-(2-phenylacetamido)-4-(2,4,5-trifluorophenyl)butanoic acid (R-IIa and (S)-3-amino-4-(2,4,5-trifluorophenyl)butanoic acid (S-IIIa)

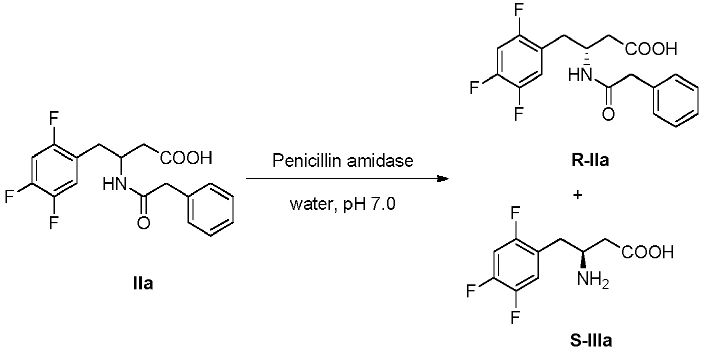

Liophylised *E. coli* Penicillin G amidase (70 µL; 66 U) is dissolved in a pH 7 solution of the **IIa** (0.2 g; 0.00057 mol) in 1 M phosphate buffer (5 mL). The reaction mixture is stirred at 25°C for 2 h (conversion 47% determined by HPLC). Then 2M HCl is added until pH 2. The mixture is extracted twice with ethyl acetate, the combined organic phases are dried and evaporated to afford 0.12 g of white residue, which contained 0.09 g of highly enantiomerically enriched (ee 95%) **(R)-IIa** and of phenylacetic acid. This residue is processed further without purification, optionally it can be recrystallized from ethyl acetate/hexane mixture to afford crystalline enantiomerically enriched product **R-IIa** (ee 95%). The water phase is neutralised to pH 7, concentrated to half volume and cooled to 4°C. The resulting precipitate is filtered and dried in an oven to afford 0.05 g of enantiomerically enriched **S-IIIa** (ee 70%).

### Example 6: (R)-3-(2-Phenylacetamido)-4-(2,4,5-trifluorophenyl)butanoic acid ((R)-IIa) and (S)-3-amino-4-(2,4,5-trifluorophenyl)butanoic acid ((S)-IIIa)

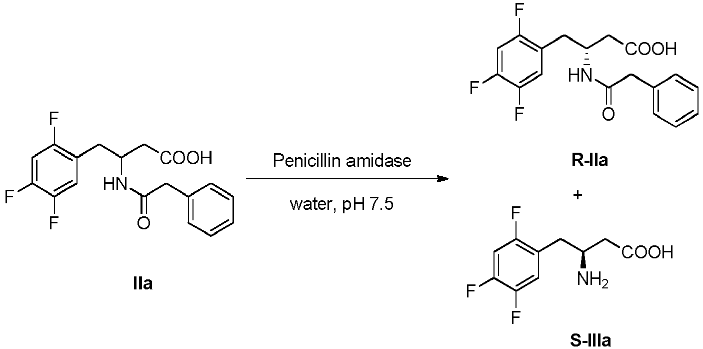

**IIa** (1.0 g; 0.00285 mol) and NaOH (0,003 mol) are dissolved in 50 mL of water and pH of the solution is adjusted to 7.5. The immobilized *Providencia rettgeri* penicillin amidase (15 g, 700 U) is added and the reaction mixture is stirred at 25°C for 50 min (conversion 62 % determined by HPLC). The enzyme is filtered off, the filtrate is acidified to pH 2 and extracted twice with ethyl acetate. The combined organic phases are washed again with water (pH 2), dried and evaporated to afford 0.49 g of white residue, which contains 0,37 g of highly enantiomerically enriched (ee 97.3%) **(R)-IIa** and 0.12 g of phenylacetic acid. The residue is processed further without additional purification. The water phase is neutralized to pH 7, concentrated to half volume and cooled to 4°C. The resulting precipitate is filtered off and dried in an oven to afford 0.25 g of enantiomerically enriched **(S)-IIIa** (ee 54%).

### Example 7: (R)-3-Amino-4-(2,4,5-trifluorophenyl)butanoic acid ((R)-IIIa)

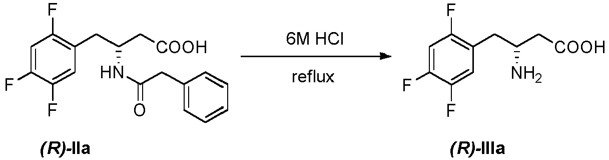

***(R)*-IIa** (1 g, 0.0043 mol, ee 99.5 %) is refluxed for 16 h in 6M HCl (50 mL). After cooling, the reaction mixture is extracted twice in ethyl acetate (50 mL). The water phase is first neutralised (pH 7), then concentrated to 10 mL and cooled to 4°C. The precipitate is filtered off and dried in an oven to afford 0.5 g of ***(R)*-IIIa** (ee 99.5%).

### Example 8: 4-(R)-(2,4,5-Trifluorobenzyl)azetidin-2-one (V)

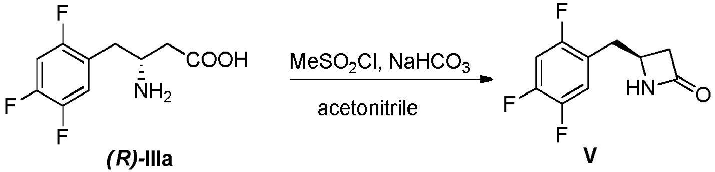

The suspension of methanesulfonyl chloride (0.88 g, 7.72 mmol), sodium bicarbonate (3.24 g, 38.6 mmol) and 3-(R)-amino-4-(2,4,5-trifluorophenyl)butanoic acid **((R)-IIIa,** 1.5 g, 6.43 mmol) in acetonitrile (50 mL), is stirred for 24 h at 120°C, after which time the fine suspension is cooled to 0°C and filtered. The filtrate is evaporated under reduced pressure to afford 1.34 g (96% yield) of 4-*(R)*-(2,4,5-trifluorobenzyl)azetidin-2-one **(V)** as pale yellow crystalline solid (ee 99.5%).

### Example 9: (R)-3-amino-1-(3-(trifluoromethyl)-5,6-dihydro-[1,2,4]triazolo[4,3-a]pyrazin-7(8H)-yl)-4-(2,4,5-trifluoro phenyl)butan-1-one (sitagliptin, Ia)

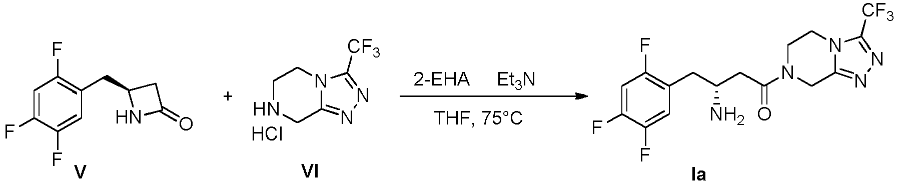

Azetidinone **V** (0.5 g, 2.32 mmol, ee 99.5 %), 3-(trifluoromethyl)-5,6,7,8-tetrahydro-[1,2,4] triazolo[4,3-a]pyrazine hydrochloride **(VI,** 1.06 g, 4.65 mmol), Et₃N (0.28 g, 4.65 mmol) and 2-ethylhexanoic acid (0.17 g, 1.16 mmol) are suspended in THF (10 mL). The resulting suspension is stirred at 75°C for 72 h. After cooling the mixture to 0°C, compound **Ia** is filtered off and the solvent is removed *in vacuo.* To the remaining oil is added water (10 mL) and the product is extracted with dichloromethane (3 × 10 mL). The organic phases are combined, dried over magnesium sulfate and concentrated *in vacuo* to give 0.90 g of sitagliptin **(Ia)** (95%, ee 99.5 %) as dark yellow oil.

### Example 10: Racemic 3-amino-4-(2,4,5-trifluorophenyl)butanoic acid (IIIa)

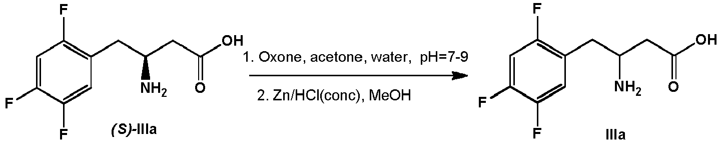

To the suspension of ***(S)*-IIIa** (2.0 g, 8.58 mmol) in acetone (35 ml) and 0.1 M phosphate buffer (35 mL), the solution of Oxone^{®} (15.8 g, 25.7 mmol) in water (20 mL) is added slowly over period of 20 min at 5°C. The pH 7-8 is maintained by addition of 5M KOH. After all Oxone^{®} is added and pH stabilized at 8.0 the mixture is allowed to warm to room temperature. After 1 hour the pH of the reaction mixture is elevated to 9.0 and the mixture is stirred for 16 hours at room temperature. Then the acetone is evaporated in vacuum and the pH of remaining water suspension is lowered to 2.0 and washed with ethyl acetate (80 mL). After the removal of water phase, the organic phase is dried over MgSO₄, filtered and evaporated in vacuum. The remaining yellow oily residue (1.9 g) is dissolved in 120 mL of methanol and cooled to 0°C. Powdered zinc (12 g) and 36% HCl (10 mL) are added and after 1 hour the mixture is allowed to stir at room temperature for further 16 hours. Zinc particles are removed by filtration and methanol is removed under reduced pressure. To the remaining oily residue, water is added and pH elevated to 8. The precipitated white solid is filtered off and dried in an oven to afford 1.9 g of **IIIa.**

## Claims

1. A process for the preparation of a compound of Formula *(R)*-II: 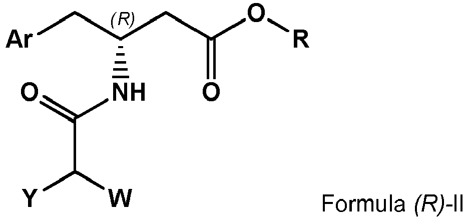 wherein
Ar is unsubstituted or substituted C₆-C₁₂-aryl, R is H or C₁-C₄-alkyl, and
W denotes unsubstituted or substituted C₆-C₁₂-aryl, furanyl or thienyl and Y independently denotes hydrogen, hydroxy, amino, chloro, bromo or methyl, or
W denotes unsubstituted or substituted C₆-C₁₂-aryloxy or C₅-C₁₂-heterocycle or -heteroaryl, and Y is hydrogen,
the process comprising:
(i) N-acylation of γ-aryl-β-aminobutyric acid or acid derivative of Formula-III, 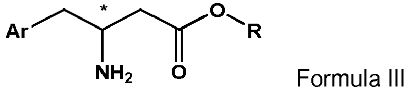 wherein Ar and R are defined as above
with an activated acid derivative of the compound of Formula IV 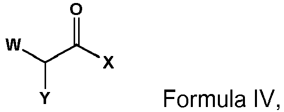 wherein Y and W are defined as above and X is a leaving group, in a solvent and a presence of a base to afford substituted acetylated γ-aryl-β-aminobutyric acid compound of Formula II 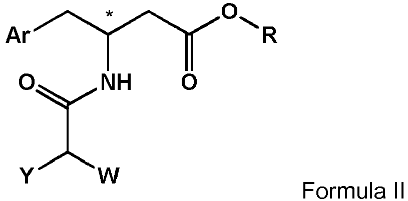
(ii) kinetic resolution of the compound of Formula II with penicillin amidase enzyme in an aqueous medium to obtain a compound of Formula II which is enantiomerically enriched in the (R)-enantiomer denoted (*R*)-II and enantiomerically enriched compound of Formula *(S)*-III 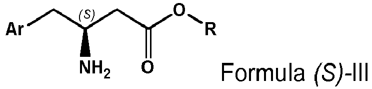 wherein Ar and R are defined as above, and
(iii) obtaining the compound of Formula *(R)*-II.

2. The process according to claim 1, wherein in step (iii) the compound of Formula *(R)*-II is obtained by acidifying the aqueous medium and extracting the compound of Formula *(R)*-II from acidic aqueous medium with a water immiscible solvent, optionally subsequently removing the water immiscible solvent.

3. The process according to claim 1 or 2, wherein Ar is halo substituted phenyl, preferably is fluoro substituted phenyl, in particular Ar is 2,4,5-trifluorophenyl.

4. The process according to any one of the preceding claims, wherein W is phenyl, and/or wherein R is H.

5. The process according to any one of the preceding claims, wherein the compound of Formula *(R)*-II obtained in step (iii) is hydrolysed to obtain a compound of Formula *(R)*-III 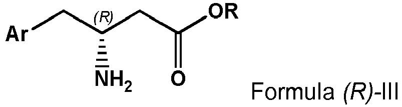 wherein Ar is as defined above in claim 1 or 3, preferably is 2,4,5-trifluorophenyl, and wherein R is as defined above in claim 1, preferably R is H.

6. The process according to claim 1, wherein enantiomerically enriched compound of Formula *(S)*-III 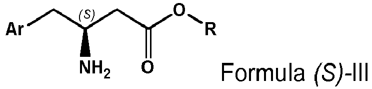 wherein Ar and R are defined as above, is used to subsequently prepare a pharmaceutically active compound.

7. The process according to any one of claims 1 to 5, wherein enantiomerically enriched compound of Formula *(R)*-III 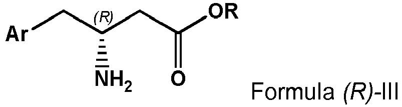 wherein Ar and R are defined as above, is used to subsequently prepare a pharmaceutically active compound, preferably a gliptin compound (DPP-4 inhibitor), in particular to prepare sitagliptin or a salt thereof.

8. The process according to any one of claims 1 to 5 and 7, wherein, after the kinetic resolution step (ii), the compound of Formula *(S)*-III in enantiomerically enriched form 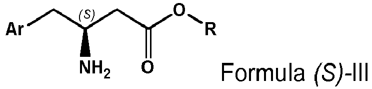 wherein Ar and R are defined as above,
is oxidized to its C-N double bonded intermediate by means of an oxidizing agent, and the thus formed C-N double bonded intermediate is reduced by means of a reducing agent, in order to obtain the compound of formula III in a racemic form, 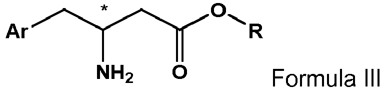 which compound of formula III in a racemic form is recycled in the process by being N-acylated according to step (i) of claim 1 in order to eventually prepare the compound of Formula *(R)*-II.

9. A process for preparing a gliptin compound (DPP-4 inhibitor) of Formula I or a salt thereof 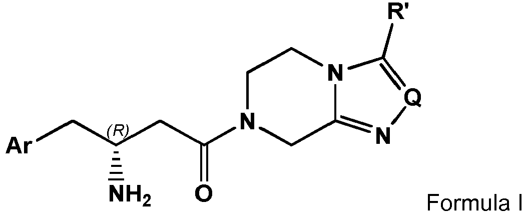 wherein Ar denotes unsubstituted or substituted C₆-C₁₂-aryl, Q denotes N, CH or a carbon substituted with unsubstituted or substituted C₁-C₆-alkyl, C₆-C₁₂-aryl or C₇-C₁₂-alkylaryl, and R' denotes H or unsubstituted or substituted C₁-C₆-alkyl, C₆-C₁₂-aryl or C₇-C₁₂-alkylaryl,
the process comprising:
(i) preparing γ-aryl-β-amino carboxylic acid of Formula *(R)*-III' 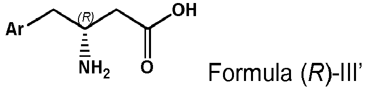 wherein Ar is defined as above,
involving a biocatalytic reaction using penicillin amidase,
(ii) cyclodehydration of the compound of Formula *(R)*-III' to obtain a β-lactam of Formula *(R)*-V 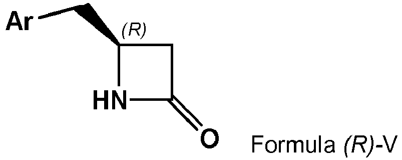 wherein Ar is defined as above,
(iii) reacting the compound of Formula (R)-V with a compound of formula VI or salt thereof, 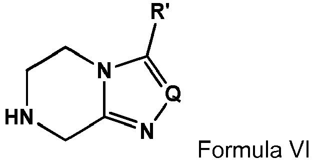 wherein R' and Q are defined as above,
in the presence of a catalyst in an organic solvent.

10. A compound of formula II 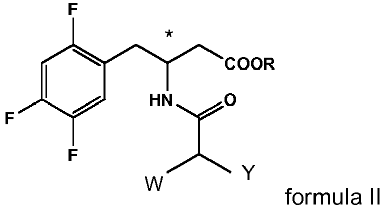 wherein R is H or C₁-C₄-alkyl,
W denotes unsubstituted or substituted C₆-C₁₂-aryl, furanyl or thienyl and Y independently denotes hydrogen, hydroxy, amino, chloro, bromo or methyl, or
W denotes unsubstituted or substituted C₆-C₁₂-aryloxy or C₅-C₁₂-heterocycle or -heteroaryl, and Y is hydrogen, wherein the compound of formula II is in racemic form or is enantiomerically enriched in either (R)- or (S)-form.

11. The compound according to claim 10, wherein W is phenyl and Y is H.

12. 4-(2,4,5-trifluorophenyl)-3-phenylacetylaminobutyric acid of Formula IIa 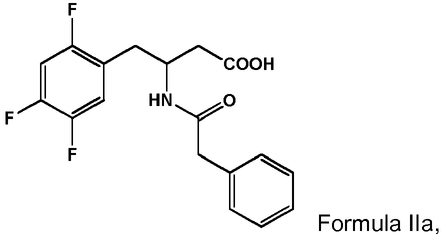 preferably enantiomerically enriched and more preferably in enantiopure form of Formula *(R)*-IIa: 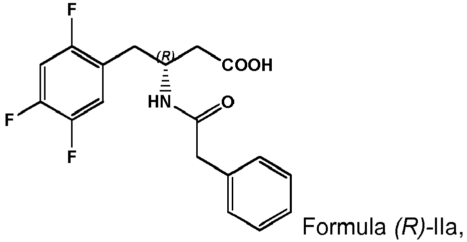 or enantiomerically enriched and more preferably in enantiopure form of Formula *(S)*-IIa:

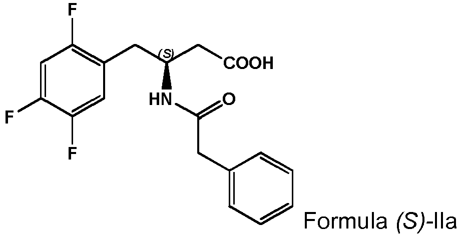

13. Use of a compound as set forth in any one of claims 10 to 12 in a process for preparing a gliptin compound.

14. A process for preparing a pharmaceutical composition comprising a gliptin compound of Formula I or a pharmaceutically acceptable salt thereof 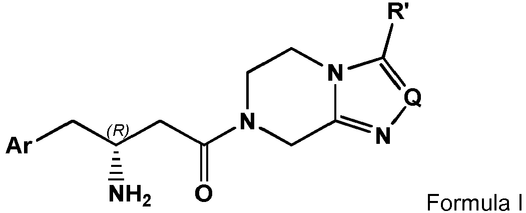 wherein Ar denotes unsubstituted or substituted C₆-C₁₂-aryl, Q denotes N, CH or a carbon substituted with unsubstituted or substituted C₁-C₆-alkyl, C₆-C₁₂-aryl or C₇-C₁₂-alkylaryl, and R' denotes H or unsubstituted or substituted C₁-C₆-alkyl, C₆-C₁₂-aryl or C₇-C₁₂-alkylaryl,
the process comprising:
carrying out a process according to claim 7 or 9 for preparing said gliptin compound of Formula I or a pharmaceutically acceptable salt thereof, and
mixing the prepared gliptin compound of Formula I or a pharmaceutically acceptable salt thereof with at least one pharmaceutically acceptable excipient or carrier to obtain a pharmaceutical composition.

15. The process according to claim 14, wherein said gliptin compound or a pharmaceutically acceptable salt thereof is combined with another pharmaceutically active ingredient, either within the same pharmaceutical composition or another pharmaceutical composition, wherein said another pharmaceutically active ingredient is selected from the group consisting of insulin sensitizers, insulin, insulin mimetics, sulfonylureas, (α-glucosidase inhibitors, glucagon receptor antagonists, GLP-1, GLP-1 analogues, GLP-1 mimetics, GLP-1 receptor agonists, GIP, GIP mimetics, PACAP, PACAP mimetics, PACAP receptor agonists, cholesterol lowering agents, PPAR-δ agonists, anti-obesity compounds, ileal bile acid transporter inhibitors, agents intended for use in inflammatory conditions, antihypertensive agents, glucokinase activators (GKAs), inhibitors of 11 (-hydroxysteroid dehydrogenase type 1, inhibitors of cholesteryl ester transfer protein (CETP) and inhibitors of fructose 1,6-bisphosphatase.

## Patentansprüche

1. Ein Verfahren zur Herstellung einer Verbindung der Formel *(R)*-II: 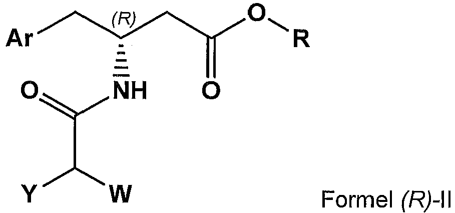 wobei
Ar unsubstituiertes oder substituiertes C₆-C₁₂-Aryl ist, R H oder C₁-C₄-Alkyl ist, und
W unsubstituiertes oder substituiertes C₆-C₁₂-Aryl, Furanyl oder Thienyl bedeutet und Y unabhängig Wasserstoff, Hydroxy, Amino, Chlor, Brom oder Methyl bedeutet oder
W unsubstituiertes oder substituiertes C₆-C₁₂-Aryloxy oder C₅-C₁₂-Heterocyclus oder -Heteroaryl bedeutet, und Y Wasserstoff ist,
wobei das Verfahren umfasst:
(i) N-Acylierung von γ-Aryl-β-Aminobuttersäure oder einem Säurederivat der Formel-III, 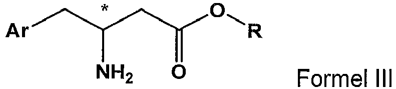 wobei Ar und R wie oben definiert sind
mit einem aktivierten Säurederivat der Verbindung der Formel IV 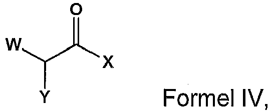 wobei Y und W wie oben definiert sind und X eine Abgangsgruppe ist, in einem Lösungsmittel und einer Anwesenheit einer Base, um eine substituierte acetylierte γ-Aryl-β-aminobuttersäure-Verbindung der Formel II zu ergeben 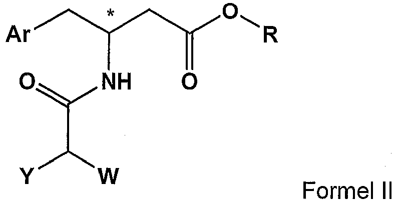
(ii) kinetische Racematspaltung der Verbindung der Formel II mit Penicillin-Amidase-Enzym in einem wässrigen Medium um eine Verbindung der Formel II, welche in dem als (*R*)-II bezeichneten (R)-Enantiomer enantiomerenangereichert ist, und eine enantiomerenangereicherte Verbindung der Formel *(S)*-III 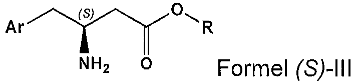 zu erhalten, wobei Ar und R wie oben definiert sind, und
(iii) Erhalten der Verbindung der Formel *(R)*-II.

2. Das Verfahren gemäß Anspruch 1, wobei in Schritt (iii) die Verbindung der Formel *(R)*-II durch Ansäuern des wässrigen Mediums und Extrahieren der Verbindung der Formel *(R)*-II aus dem sauren wässrigen Medium mit einem wasserunlöslichen Lösungsmittel erhalten wird, optional nach Entfernen des wasserunlöslichen Lösungsmittels.

3. Verfahren gemäß Anspruch 1 oder 2, wobei Ar halogensubstituiertes Phenyl ist, bevorzugt fluorsubstituiertes Phenyl, wobei Ar insbesondere 2,4,5-Trifluorphenyl ist.

4. Das Verfahren gemäß einem der vorstehenden Ansprüche, wobei W Phenyl ist und/oder wobei R H ist.

5. Das Verfahren gemäß einem der vorstehenden Ansprüche, wobei die in Schritt (iii) erhaltene Verbindung der Formel *(R)*-II hydrolysiert wird, um eine Verbindung der Formel *(R)*-III 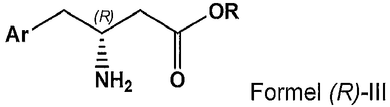 zu erhalten, wobei Ar wie oben in Anspruch 1 oder 3 definiert, bevorzugt 2,4,5-Trifluorphenyl, ist, und wobei R wie oben in Anspruch 1 definiert, bevorzugt H, ist.

6. Das Verfahren gemäß Anspruch 1, wobei die enantiomerenangereicherte Verbindung der Formel *(S)*-III 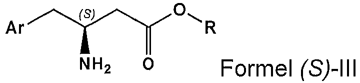 wobei Ar und R wie oben definiert sind, verwendet wird, um nachfolgend eine pharmazeutisch aktive Verbindung herzustellen.

7. Das Verfahren gemäß einem der Ansprüche 1 bis 5, wobei die enantiomerenangereicherte Verbindung der Formel *(R)*-III 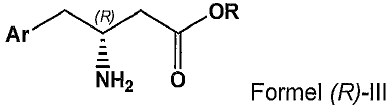 wobei Ar und R wie oben definiert sind, verwendet wird, um nachfolgend eine pharmazeutisch aktive Verbindung, bevorzugt eine Gliptinverbindung (DPP-4-Inhibitor), herzustellen, insbesondere um Sitagliptin oder ein Salz davon herzustellen.

8. Das Verfahren gemäß einem der Ansprüche 1 bis 5 und 7, wobei nach dem kinetischen Racematspaltungsschritt (ii) die Verbindung der Formel *(S)*-III in enantiomerenangereicherter Form 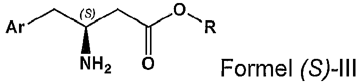 wobei Ar und R wie oben definiert sind,
mittels eines Oxidationsmittels zu ihrem C-N-Doppelbindungs-Intermediat oxidiert wird, und das so gebildete C-N-Doppelbindungs-Intermediat mittels eines Reduktionsmittels reduziert wird, um die Verbindung der Formel III in einer racemischen Form zu erhalten, 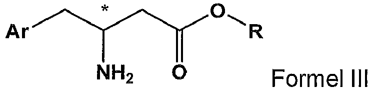 wobei die Verbindung der Formel III in einer recemischen Form in das Verfahren zurückgeführt wird, indem sie gemäß Schritt (i) des Anspruchs 1 N-acyliert wird, um schließlich die Verbindung der Formel (*R*)-II herzustellen.

9. Ein Verfahren zum Herstellen einer Gliptinverbindung (DPP-4-Inhibitor) der Formel I oder eines Salzes davon 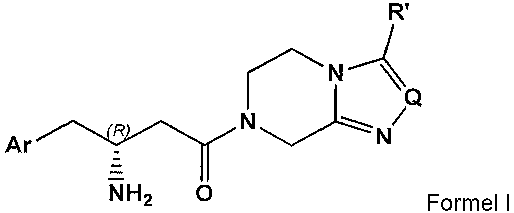 wobei Ar unsubstituiertes oder substituiertes C₆-C₁₂-Aryl bedeutet, Q N, CH oder einen mit unsubstituiertem oder substituiertem C₁-C₆-Alkyl, C₆-C₁₂-Aryl oder C₇-C₁₂-Alkylaryl substituierten Kohlenstoff bedeutet und R' H oder unsubstituiertes oder substituiertes C₁-C₆-Alkyl, C₆-C₁₂-Aryl oder C₇-C₁₂-Alkylaryl bedeutet,
wobei das Verfahren umfasst:
(i) Herstellen von γ-Aryl-β-aminocarbonsäure der Formel *(R)*-III' 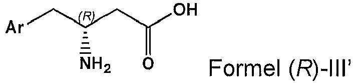 wobei Ar wie oben definiert ist,
unter Einbeziehung einer biokatalytischen Reaktion unter Verwendung von Penicillin-Amidase,
(ii) Cyclodehydratisierung der Verbindung der Formel *(R)*-III', um ein β-Lactam der Formel *(R)*-V 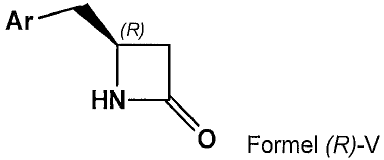 zu erhalten, wobei Ar wie oben definiert ist,
(iii) Umsetzen der Verbindung der Formel (R)-V mit einer Verbindung der Formel VI oder einem Salz davon, 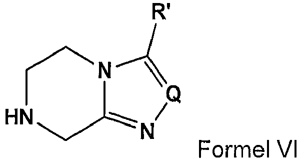 wobei R' und Q wie oben definiert sind,
in Gegenwart eines Katalysators in einem organischen Lösungsmittel.

10. Eine Verbindung der Formel II 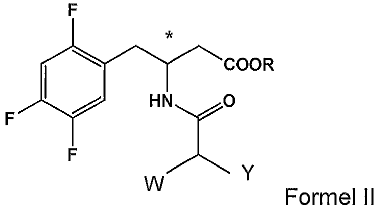 wobei R H oder C₁-C₄-Alkyl ist,
W unsubstituiertes oder substituiertes C₆-C₁₂-Aryl, Furanyl oder Thienyl bedeutet und Y unabhängig Wasserstoff, Hydroxy, Amino, Chlor, Brom oder Methyl bedeutet oder W unsubstituiertes oder substituiertes C₆-C₁₂-Aryloxy oder C₅-C₁₂-Heterocyclus oder -Heteroaryl bedeutet, und Y Wasserstoff ist, wobei die Verbindung der Formel II in racemischer Form ist oder entweder in der (R)- oder der (S)-Form enantiomerenangereichert ist.

11. Die Verbindung gemäß Anspruch 10, wobei W Phenyl und Y H ist.

12. 4-(2,4,5-Trifluorophenyl)-3-phenylacetylaminobuttersäure der Formel IIa 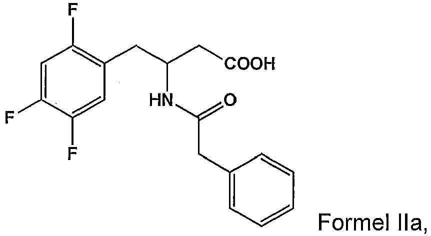 bevorzugt enantiomerenangereichert und mehr bevorzugt in enantiomerenreiner Form der Formel *(R)*-IIa: 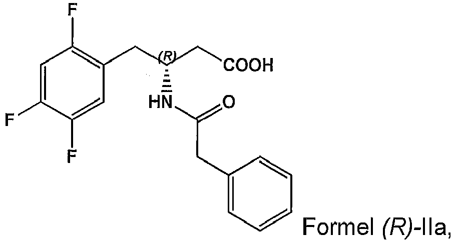 oder enantiomerenangereichert und mehr bevorzugt in enantiomerenreiner Form der Formel *(S)*-IIa:

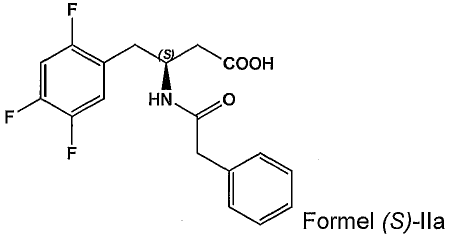

13. Verwendung einer Verbindung wie dargelegt in einem der Ansprüche 10 bis 12 in einem Verfahren zur Herstellung einer Gliptinverbindung.

14. Ein Verfahren zur Herstellung einer pharmazeutischen Zusammensetzung umfassend eine Gliptinverbindung der Formel I oder eines pharmazeutisch akzeptierbaren Salzes davon 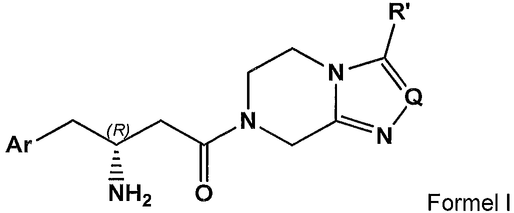 wobei Ar unsubstituiertes oder substituiertes C₆-C₁₂-Aryl bedeutet, Q N, CH oder einen mit unsubstituiertem oder substituiertem C₁-C₆-Alkyl, C₆-C₁₂-Aryl oder C₇-C₁₂-Alkylaryl substituierten Kohlenstoff bedeutet und R' H oder unsubstituiertes oder substituiertes C₁-C₆-Alkyl, C₆-C₁₂-Aryl oder C₇-C₁₂-Alkylaryl bedeutet,
wobei das Verfahren umfasst:
Ausführen eines Verfahrens gemäß Anspruch 7 oder 9 zum Herstellen der Gliptinverbindung der Formel I oder eines pharmazeutisch akzeptierbaren Salzes davon, und
Mischen der hergestellten Gliptinverbindung der Formel I oder eines pharmazeutisch akzeptierbaren Salzes davon mit zumindest einem pharmazeutisch akzeptierbaren Hilfsstoff oder Träger, um eine pharmazeutische Zusammensetzung zu erhalten.

15. Das Verfahren gemäß Anspruch 14, wobei die Gliptinverbindung oder ein pharmazeutisch akzeptierbares Salz davon mit einem anderen pharmazeutisch aktiven Wirkstoff, entweder innerhalb derselben oder einer anderen pharmazeutischen Zusammensetzung, kombiniert wird, wobei der andere pharmazeutisch aktive Wirkstoff ausgewählt wird aus der Gruppe bestehend aus Insulin-Sensitizern, Insulin, Insulin-Mimetika, Sulfonylharnstoffen, α-Glucosidase-Inhibitoren, Glucagonrezeptor-Antagonisten, GLP-1, GLP-1-Analoga, GLP-1-Mimetika, GLP-1-Rezeptor-Agonisten, GIP, GIP-Mimetika, PACAP, PACAP-Mimetika, PACAP-Rezeptor-Agonisten, cholesterinsenkende Wirkstoffe, PPAR-δ-Agonisten, Verbindungen gegen Adipositas, ileale Gallensäuretranporter-Inhibitoren, für die Verwendung bei Entzündungszuständen bestimmte Wirkstoffe, antihypertensive Wirkstoffe, Glucokinase-Aktivatoren (GKAs), Inhibitoren der 11-Hydroxysteroid-Dehydrogenase vom Typ 1, Inhibitoren des Cholesterylester-Transferproteins (CETP) und Inhibitoren der Fructose-1,6-Bisphosphatase.

## Revendications

1. Procédé de préparation d'un composé de formule (*R*)-II : 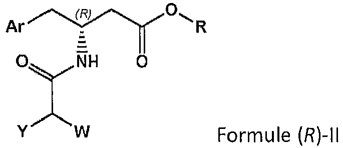 dans laquelle
Ar est un groupe aryle en C₆-C₁₂ non substitué ou substitué, R est H ou un groupe alkyle en C₁-C₄, et
W désigne un groupe aryle en C₆-C₁₂, furanyle ou thiényle non substitué ou substitué et Y désigne indépendamment un atome d'hydrogène, un groupe hydroxy, amino, chloro, bromo ou méthyle, ou
W désigne un groupe aryloxy en C₆-C₁₂ ou hétérocycle ou hétéroaryle en C₅-C₁₂ non substitué ou substitué, et Y est un atome d'hydrogène,
le procédé comprenant :
(i) la N-acylation d'un acide γ-aryl-β-aminobutyrique ou d'un dérivé d'acide de formule III, 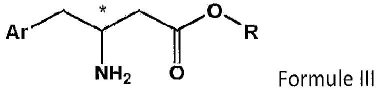 dans laquelle Ar et R sont tels que définis ci-dessus
avec un dérivé d'acide activé du composé de formule IV 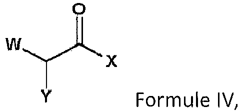 dans laquelle Y et W sont tels que définis ci-dessus et X est un groupe partant, dans un solvant et en présence d'une base pour obtenir un composé d'acide γ-aryl-β-aminobutyrique acylé substitué de formule II 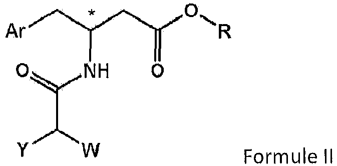
(ii) la résolution cinétique du composé de formule II avec une enzyme pénicilline amidase dans un milieu aqueux pour obtenir un composé de formule II qui est enrichi sur le plan énantiomère en l'énantiomère (*R*) appelé (*R*)-II et un composé de formule (*S*)-III enrichi sur le plan énantiomère 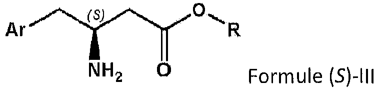 dans laquelle Ar et R sont tels que définis ci-dessus, et
(iii) l'obtention du composé de formule (*R*)-II.

2. Procédé selon la revendication 1, dans lequel dans l'étape (iii) le composé de formule (*R*)-II est obtenu par acidification du milieu aqueux et par extraction du composé de formule (*R*)-II hors du milieu aqueux acide avec un solvant non miscible avec l'eau, éventuellement par élimination ultérieure du solvant non miscible avec l'eau.

3. Procédé selon la revendication 1 ou 2, dans lequel Ar est un groupe phényle halogéno-substitué, de préférence phényle fluoro-substitué, en particulier Ar est le groupe 2,4,5-trifluorophényle.

4. Procédé selon l'une quelconque des revendications précédentes, dans lequel W est un groupe phényle, et/ou dans lequel R est H.

5. Procédé selon l'une quelconque des revendications précédentes, dans lequel le composé de formule (*R*)-II obtenu dans l'étape (iii) est hydrolysé pour obtenir un composé de formule (*R*)-III 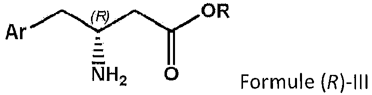 dans laquelle Ar est tel que défini ci-dessus dans la revendication 1 ou 3, de préférence est le groupe 2,4,5-trifluorophényle, et dans laquelle R est tel que défini ci-dessus dans la revendication 1, de préférence R est H.

6. Procédé selon la revendication 1, dans lequel le composé de formule (*S*)-III enrichi sur le plan énantiomère 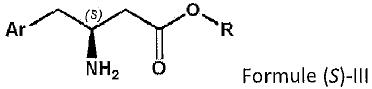 dans laquelle Ar et R sont tels que définis ci-dessus, est utilisé pour préparer ultérieurement un composé pharmaceutiquement actif.

7. Procédé selon l'une quelconque des revendications 1 à 5, dans lequel le composé de formule (*R*)-III enrichi sur le plan énantiomère 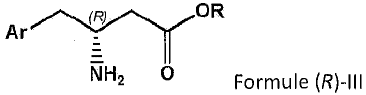 dans laquelle Ar et R sont tels que définis ci-dessus, est utilisé pour préparer ultérieurement un composé pharmaceutiquement actif, de préférence un composé de gliptine (inhibiteur de DPP-4), en particulier pour préparer la sitagliptine ou un sel de celle-ci.

8. Procédé selon l'une quelconque des revendications 1 à 5 et 7, dans lequel, après l'étape de résolution cinétique (ii), le composé de formule (*S*)-III sous une forme enrichie sur le plan énantiomère 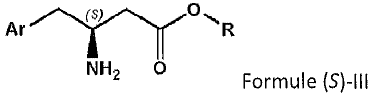 dans laquelle Ar et R sont tels que définis ci-dessus,
est oxydé en son intermédiaire à double liaison C-N au moyen d'un agent d'oxydation, et l'intermédiaire à double liaison C-N ainsi formé est réduit au moyen d'un agent réducteur, pour obtenir le composé de formule III sous une forme racémique, 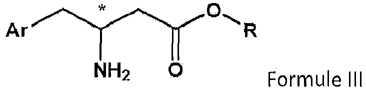 lequel composé de formule III sous forme racémique est recyclé dans le procédé par une N-acylation selon l'étape (i) de la revendication 1 pour préparer à terme le composé de formule (*R*)-II.

9. Procédé de préparation d'un composé de gliptine (inhibiteur de DPP-4) de formule I ou sel de celui-ci 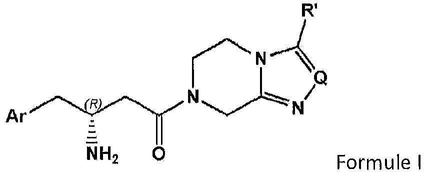 dans laquelle Ar désigne un groupe aryle en C₆-C₁₂ non substitué ou substitué, Q désigne N, CH ou un atome de carbone substitué par un groupe alkyle en C₁-C₆, aryle en C₆-C₁₂ ou alkylaryle en C₇-C₁₂ non substitué ou substitué, et R' désigne H ou un groupe alkyle en C₁-C₆, aryle en C₆-C₁₂ ou alkylaryle en C₇-C₁₂ non substitué ou substitué,
le procédé comprenant :
(i) la préparation d'un acide γ-aryl-β-aminocarboxylique de formule (*R*)-III' 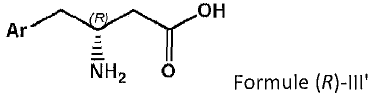 dans laquelle Ar est tel que défini ci-dessus,
mettant en jeu une réaction biocatalytique utilisant de la pénicilline amidase,
(ii) la cyclodéshydratation du composé de formule (*R*)-III' pour obtenir un β-lactame de formule (*R*)-V 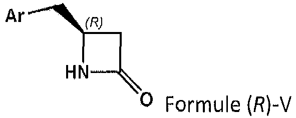 dans laquelle Ar est tel que défini ci-dessus,
(iii) la réaction du composé de formule (*R*)-V avec un composé de formule VI ou un sel de celui-ci 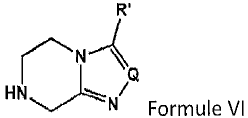 dans laquelle R' et Q sont tels que définis ci-dessus,
en présence d'un catalyseur dans un solvant organique.

10. Composé de formule II 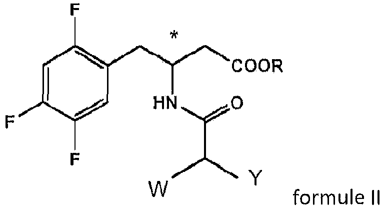 dans laquelle R est H ou un groupe alkyle en C₁-C₄,
W désigne un groupe aryle en C₆-C₁₂, furanyle ou thiényle non substitué ou substitué et Y désigne indépendamment un atome d'hydrogène, un groupe hydroxy, amino, chloro, bromo ou méthyle, ou
W désigne un groupe aryloxy en C₆-C₁₂ ou hétérocycle ou hétéroaryle en C₅-C₁₂ non substitué ou substitué, et Y est un atome d'hydrogène, dans lequel le composé de formule II est sous une forme racémique ou est enrichi sur le plan énantiomère sous forme (*R*) ou (*S*).

11. Composé selon la revendication 10, dans lequel W est un groupe phényle et Y est H.

12. Acide 4-(2,4,5-trifluorophényl)-3-phénylacétylaminobutyrique de formule IIa 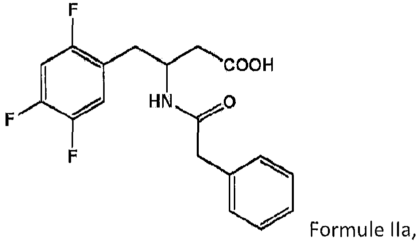 de préférence enrichi sur le plan énantiomère et de manière davantage préférée sous une forme pure sur le plan énantiomère de formule (*R*)-IIa : 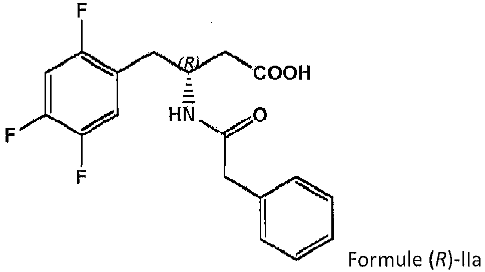 ou enrichi sur le plan énantiomère et de manière davantage préférée sous une forme pure sur le plan énantiomère de formule (*S*)-IIa :

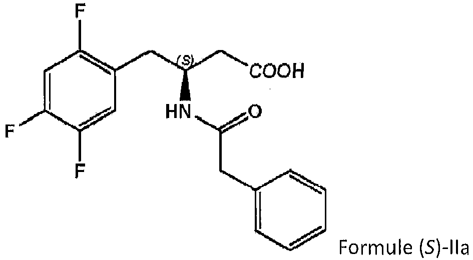

13. Utilisation d'un composé tel que présenté dans l'une quelconque des revendications 10 à 12 dans un procédé de préparation d'un composé de gliptine.

14. Procédé de préparation d'une composition pharmaceutique comprenant un composé de gliptine de formule I ou un sel pharmaceutiquement acceptable de celui-ci 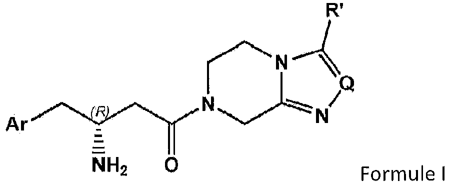 dans laquelle Ar désigne un groupe aryle en C₆-C₁₂ non substitué ou substitué, Q désigne N, CH ou un atome de carbone substitué par un groupe alkyle en C₁-C₆, aryle en C₆-C₁₂ ou alkylaryle en C₇-C₁₂ non substitué ou substitué, et R' désigne H ou un groupe alkyle en C₁-C₆, aryle en C₆-C₁₂ ou alkylaryle en C₇-C₁₂ non substitué ou substitué,
le procédé comprenant :
la réalisation d'un procédé selon la revendication 7 ou 9 pour la préparation dudit composé de gliptine de formule I ou d'un sel pharmaceutiquement acceptable de celui-ci, et
le mélange du composé de gliptine de formule 1 préparé ou d'un sel pharmaceutiquement acceptable de celui-ci avec au moins un excipient ou véhicule pharmaceutiquement acceptable pour obtenir une composition pharmaceutique.

15. Procédé selon la revendication 14, dans lequel ledit composé de gliptine ou un sel pharmaceutiquement acceptable de celui-ci est combiné avec un autre ingrédient pharmaceutiquement actif, dans la même composition pharmaceutique ou dans une autre composition pharmaceutique, dans lequel ledit autre ingrédient pharmaceutiquement actif est choisi dans le groupe constitué des sensibilisants à l'insuline, de l'insuline, des produits imitant l'insuline, des sulfonylurées, des inhibiteurs d'α-glucosidase, des antagonistes du récepteur de glucagon, du GLP-1, des analogues de GLP-1, des produits imitant le GLP-1, des agonistes du récepteur de GLP-1, du GIP, des produits imitant le GIP, du PACAP, des produits imitant le PACAP, des agonistes du récepteur de PACAP, des agents réduisant le cholestérol, des agonistes de PPAR-δ, des composés anti-obésité, des inhibiteurs du transporteur de l'acide biliaire iléal, des agents destinés à une utilisation dans des états inflammatoires, des agents antihypertenseurs, des activateurs de la glucokinase (GKA), des inhibiteurs de la 11-hydroxystéroïde déshydrogénase de type 1, des inhibiteurs de la protéine de transfert du cholestérylester (CETP) et des inhibiteurs de la fructose 1,6-bisphosphatase.
